# EUROPEAN PATENT SPECIFICATION

(11) **EP 0 843 551 B1**
(45) Date of publication and mention of the grant of the patent: **06.02.2002**
(21) Application number: 96926243.5
(22) Date of filing: 02.08.1996
(51) Int. Cl.: A61K 31/415, C07D 405/02, C07D 207/34, C07D 405/04, A01N 43/56

(54) **ENDOTHELIN RECEPTOR ANTAGONISTS**
ENDOTHELINREZEPTORANTAGONISTEN
ANTAGONISTES DU RECEPTEUR DE L'ENDOTHELINE

(30) Priority: 02.08.1995 US 1792; 01.02.1996 US 10982
(43) Date of publication of application: 27.05.1998
(73) Proprietor: SMITHKLINE BEECHAM CORPORATION, Philadelphia Pennsylvania 19101 (US)
(72) Inventor: LUENGO, Juan Ignacio, Audubon, PA 19403 (US); ELLIOTT, John, Duncan, Wayne, PA 19087 (US); XIANG, Jia-Ning, Wayne, PA 19087 (US)
(74) Representative: Florence, Julia Anne
(86) International application number: PCT/US96/12661
(87) International publication number: WO 97/04773

(56) References cited:
- EP-A- 0 714 897
- US-A- 3 703 513
- US-A- 4 965 282

## Description

The present invention relates to novel pyrroles, pyrazoles and triazoles, pharmaceutical compositions containing these compounds and their use as endothelin receptor antagonists.

Endothelin (ET) is a highly potent vasoconstrictor peptide synthesized and released by the vascular endothelium. Endothelin exists as three isoforms, ET- 1, ET-2 and ET-3. [Unless otherwise stated "endothelin" shall mean any or all of the isoforms of endothelin]. Endothelin has profound effects on the cardiovascular system, and in particular, the coronary, renal and cerebral circulation. Elevated or abnormal release of endothelin is associated with smooth muscle contraction which is involved in the pathogenesis of cardiovascular, cerebrovascular, respiratory and renal pathophysiology. Elevated levels of endothelin have been reported in plasma from patients with essential hypertension, acute myocardial infarction, subarachnoid hemorrhage, atherosclerosis, and patients with uraemia undergoing dialysis.

In vivo, endothelin has pronounced effects on blood pressure and cardiac output. An intravenous bolus injection of ET (0.1 to 3 nmol/kg) in rats causes a transient, dose-related depressor response (lasting 0.5 to 2 minutes) followed by a sustained, dose-dependent rise in arterial blood pressure which can remain elevated for 2 to 3 hours following dosing. Doses above 3 nmol/kg in a rat often prove fatal.

Endothelin appears to produce a preferential effect in the renal vascular bed. It produces a marked, long-lasting decrease in renal blood flow, accompanied by a significant decrease in GFR, urine volume, urinary sodium and potassium excretion. Endothelin produces a sustained antinatriuretic effect, despite significant elevations in atrial natriuretic peptide. Endothelin also stimulates plasma renin activity. These findings suggest that ET is involved in the regulation of renal function and is involved in a variety of renal disorders including acute renal failure, cyclosporine nephrotoxicity, radio contrast induced renal failure and chronic renal failure.

Studies have shown that in vivo, the cerebral vasculature is highly sensitive to both the vasodilator and vasoconstrictor effects of endothelin. Therefore, ET may be an important mediator of cerebral vasospasm, a frequent and often fatal consequence of subarachnoid hemorrhage.

ET also exhibits direct central nervous system effects such as severe apnea and ischemic lesions which suggests that ET may contribute to the development of cerebral infarcts and neuronal death.

ET has also been implicated in myocardial ischemia (Nichols et al. Br. J. Pharm. 99: 597-601, 1989 and Clozel and Clozel, Circ. Res., 65: 1193-1200, 1989) coronary vasospasm (Fukuda et al., Eur. J. Pharm. 165: 301-304, 1989 and Lüscher, Circ. 83: 701, 1991) heart failure, proliferation of vascular smooth muscle cells, (Takagi, Biochem & Biophys. Res. Commun.; 168: 537-543, 1990, Bobek et al., Am. J. Physiol. 258:408-C415, 1990) and atherosclerosis, (Nakaki et al., Biochem. & Biophys. Res. Commun. 158: 880-881, 1989, and Lerman et al., New Eng. J. of Med. 325: 997-1001, 1991). Increased levels of endothelin have been shown after coronary balloon angioplasty (Kadel et al., No. 2491 Circ. 82: 627, 1990).

Further, endothelin has been found to be a potent constrictor of isolated mammalian airway tissue including human bronchus (Uchida et al., Eur J. of Pharm. 154: 227-228 1988, LaGente, Clin. Exp. Allergy 20: 343-348, 1990; and Springall et al., Lancet, 337: 697-701, 1991). Endothelin may play a role in the pathogenesis of interstitial pulmonary fibrosis and associated pulmonary hypertension, Glard et al., Third International Conference on Endothelin, 1993, p. 34 and ARDS (Adult Respiratory Distress Syndrome), Sanai et al., Supra, p. 112.

Endothelin has been associated with the induction of hemorrhagic and necrotic damage in the gastric mucosa (Whittle et al., Br. J. Pharm. 95: 1011-1013, 1988); Raynaud's phenomenon, Cinniniello et al., Lancet 337: 114-115, 1991); Crohn's Disease and ulcerative colitis, Munch et al., Lancet, Vol. 339, p. 381; Migraine (Edmeads, Headache, Feb. 1991 p 127); Sepsis (Weitzberg et al., Circ. Shock 33: 222-227, 1991; Pittet et al., Ann. Surg. 213: 262-264, 1991), Cyclosporin-induced renal failure or hypertension (Eur. J. Pharmacol., 180: 191-192, 1990, Kidney Int, 37: 1487-1491, 1990) and endotoxin shock and other endotoxin induced diseases (Biochem. Biophys. Res. Commun., 161: 1220-1227, 1989, Acta Physiol. Scand. 137: 317-318, 1989) and inflammatory skin diseases. (Clin Res. 41:451 and 484, 1993).

Endothelin has also been implicated in preclampsia of pregnancy. Clark et al., Am. J. Obstet. Gynecol. March 1992, p. 962-968; Kamor et al., N. Eng. J. of Med., Nov 22, 1990, p. 1486-1487; Dekker et al., Eur J. Ob. and Gyn. and Rep. Bio. 40 (1991) 215-220; Schiff et al., Am. J. Ostet. Gynecol. Feb 1992, p. 624-628; diabetes mellitus, Takahashi et al., Diabetologia (1990) 33:306-310; and acute vascular rejection following kidney transplant, Watschinger et al., Transplantation Vol. 52, No. 4, pp. 743-746.

Endothelin stimulates both bone resorption and anabolism and may have a role in the coupling of bone remodeling. Tatrai et al. Endocrinology, Vol. 131, p. 603-607.

Endothelin has been reported to stimulate the transport of sperm in the uterine cavity, Casey et al., J. Clin. Endo and Metabolism, Vol. 74, No. 1, p. 223-225, therefore endothelin antagonists may be useful as male contraceptives. Endothelin modulates the ovarian/menstrual cycle, Kenegsberg, J. of Clin. Endo. and Met., Vol. 74, No. 1, p. 12, and may also play a role in the regulation of penile vascular tone in man, Lau et al., Asia Pacific J. of Pharm., 1991, 6:287-292 and Tejada et al., J. Amer. Physio. Soc. 1991, H1078-H1085. Endothelin also mediates a potent contraction of human prostatic smooth muscle, Langenstroer et al., J. Urology, Vol. 149, p. 495-499.

Thus, endothelin receptor antagonists would offer a unique approach toward the pharmacotherapy of hypertension, acute and chronic renal failure, ischemia induced renal failure, sepsis-endotoxin induced renal failure, prophylaxis and/or treatment of radio-contrast induced renal failure, acute and chronic cyclosporin induced renal failure, cerebrovascular disease, cerebrovascular spasm, subarachnoid hemorrhage, myocardial ischemia, angina, congestive heart failure, acute coronary syndrome, myocardial salvage, unstable angina, asthma, primary pulmonary hypertension, pulmonary hypertension secondary to intrinsic pulmonary disease, atherosclerosis, Raynaud's phenomenon, ulcers, sepsis, migraine, glaucoma, endotoxin shock, endotoxin induced multiple organ failure or disseminated intravascular coagulation, cyclosporin-induced renal failure and as an adjunct in angioplasty for prevention of restenosis, diabetes, diabetic retinopathy, retinopathy, diabetic nephropathy, diabetic macrovascular disease, atherosclerosis, preclampsia of pregnancy, bone remodeling, kidney transplant, male contraceptives, infertility and priaprism and benign prostatic hypertrophy.

### SUMMARY OF THE INVENTION

This invention comprises compounds represented by Formula (I) and pharmaceutical compositions containing these compounds, and their use as endothelin receptor antagonists which are useful in the treatment of a variety of cardiovascular and renal diseases including but not limited to: hypertension, acute and chronic renal failure, cyclosporine induced nephrotoxicity, benign prostatic hypertrophy, pulmonary hypertension, migraine, stroke, subarachnoid hemorrhage, cerebrovascular vasospasm, myocardial ischemia, angina, congestive heart failure, atherosclerosis, diabetic nephropathy, diabetic retinopathy, retinopathy, diabetic macrovascular disease, atherosclerosis and as an adjunct in angioplasty for prevention of restenosis.

This invention further constitutes a method for antagonizing endothelin receptors in an animal, including humans, which comprises administering to an animal in need thereof an effective amount of a compound of Formula (I).

This invention also constitutes intermediates represented by Formula (II). In a further aspect the present invention provides a process for the preparation of a compound of Formula (I)(d).

### DETAILED DESCRIPTION OF THE INVENTION

The compounds of this invention are represented by structural Formula (I): 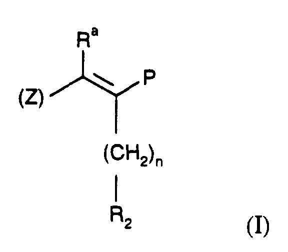 wherein (Z) is 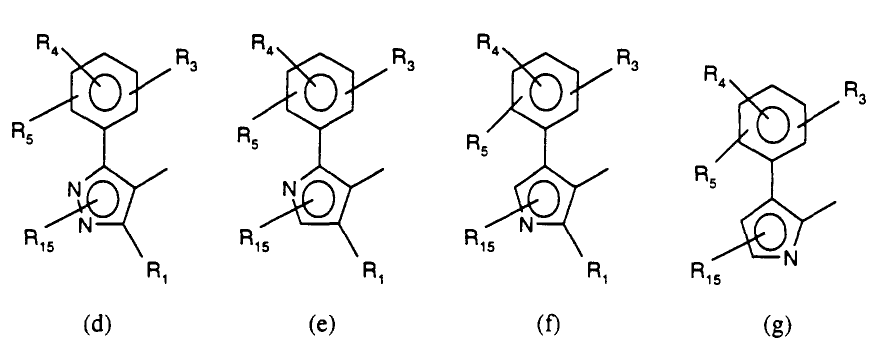 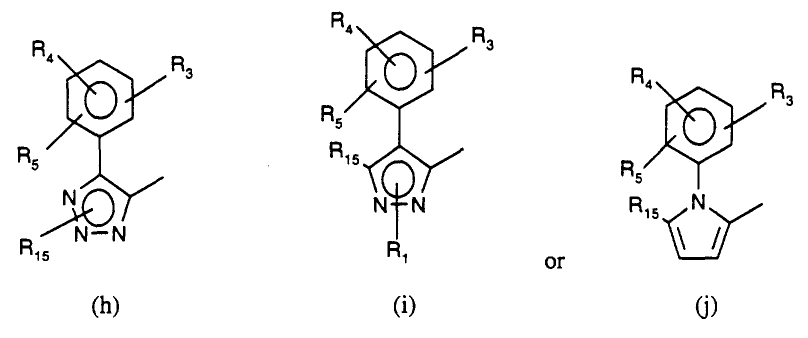
P is tetrazol-5-yl, CO₂R₆ or C(O)N(R₆)S(O)_{q}R₁₀;
R^{a} is independently hydrogen or C₁₋₆alkyl;
R₁ is independently hydrogen, Ar, C₁₋₆alkyl or C₁₋₆ alkoxy;
R₂ is Ar, C₁₋₈alkyl, C(O)R₁₄ or 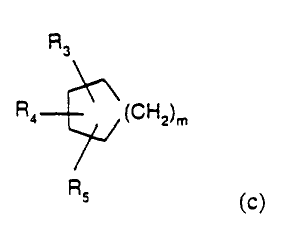
R₃ and R₅ are independently R₁₃OH, C₁₋₈alkoxy, S(O)_{q}R₁₁, N(R₆)₂, NO₂, Br, F, I, Cl, CF₃, NHCOR₆, R₁₃CO₂R₇, -X-R₉-Y, -X(C(R₆)₂)OR₆, -(CH₂)ₘX'R₈ or-X(CH₂)ₙR₈ wherein each methylene group within -X(CH₂)ₙR₈ may be unsubstituted or substituted by one or two -(CH₂)ₙAr groups;
R₄ is independently R₁₁, OH, C₁₋₅alkoxy, S(O)_{q}R₁₁, N(R₆)₂, Br, F, I, Cl or NHCOR₆, wherein the C₁₋₅alkoxy may be unsubstituted or substituted by OH, methoxy or halogen;
R₆ is independently hydrogen or C₁₋₈alkyl;
R₇ is independently hydrogen, C₁₋₁₀alkyl, C₂₋₁₀alkenyl or C₂₋₈alkynyl, all of which may be unsubstituted or substituted by one or more OH, N(R₆)₂, CO₂R₁₂, halogen or XC₁₋₁₀alkyl; or R₇ is (CH₂)ₙAr;
R₈ is independently R₁₁, CO₂R₇, CO₂C(R₁₁)₂O(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, CN, CO₂(CH₂)ₘC(O)N(R₆)₂, C(R₁₁)₂N(R₇)₂, C(O)N(R₆)₂, NR₇C(O)NR₇SO₂R₁₁, OR₆, or tetrazolyl which is unsubstituted or substituted by C₁₋₆alkyl;
R₉ is independently a bond, C₁₋₁₀alkylene, C₁₋₁₀alkenylene, C₁₋₁₀alkylidene, C₁₋ ₁₀alkynylene, all of which may be linear or branched, or phenylene, all of which may be unsubstituted or substituted by one of more OH, N(R₆)₂, COOH or halogen;
R₁₀ is independently C₁₋₁₀alkyl, N(R₆)₂ or Ar;
R₁₁ is independently hydrogen, Ar, C₁₋₈alkyl, C₂₋₈alkenyl, C₂₋₈alkynyl, all of which may be unsubstituted or substituted by one or more OH, CH₂OH, N(R₆)₂ or halogen;
R₁₂ is independently hydrogen, C₁₋₆alkyl, C₂₋₆alkenyl or C₂₋₇alkynyl;
R₁₃ is independently divalent Ar, C₁₋₁₀alkylene, C₁₋₁₀alkylidene, C₂₋₁₀alkenylene, all of which may be unsubstituted or substituted by one or two OH, CH₂OH, N(R₆)₂ or halogen;
R₁₄ is independently hydrogen, C₁₋₁₀alkyl, XC₁₋₁₀alkyl, Ar or XAr;
R₁₅ is independently hydrogen, Ar, C₁₋₆alkyl, or XAr;
R₁₆ is independently C₁₋₆alkyl or phenyl substituted by one or more C₁₋₆alkyl, OH, C₁₋₅alkoxy, S(O)_{q}R₆, N(R₆)₂, Br, F, I, Cl, CF₃ or NHCOR₆;
X is independently (CH₂)ₙ, O, NR₆ or S(O)_{q};
X' is independently O, NR₆ or S(O)_{q};
Y is independently CH₃ or X(CH₂)ₙAr;
Ar is: 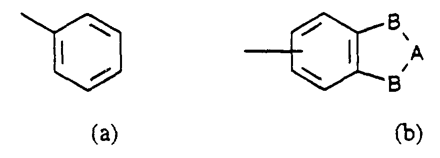 naphthyl, indolyl, pyridyl, thienyl, oxazolidinyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or two Z₁ or Z₂ groups;
A is independently C=O, or (C(R₆)₂)ₘ;
B is independently -CH₂- or -O-;
Z₁ and Z₂ are independently hydrogen, XR₆, C₁₋₈alkyl, (CH₂)_{q}CO₂R₆, C(O)N(R₆)₂, CN, (CH₂)ₙOH, NO₂, F, Cl, Br, I, N(R₆)₂, NHC(O)R₆, O(CH₂)ₘC(O)NRₐSO₂R₁₆, (CH₂)ₘOC(O)NRₐSO₂R₁₆, O(CH₂)ₘNRₐC(O)NRₐSO₂R₁₆ or tetrazolyl which may be unsubstituted or substituted by one or two C₁₋₆alkyl, CF₃ or C(O)R₆;
m is independently 1 to 3;
n is independently 0 to 6;
q is independently 0, 1 or 2;
provided R₃, R₄ and R₅ are not O-O(CH₂)ₙAr;
and further provided that R₂ is not dihydrobenzofuran;
or a pharmaceutically acceptable salt thereof.
   All alkyl, alkenyl, alkynyl and alkoxy groups may be straight or branched. Halogen may be Br, Cl, F or I.

The compounds of the present invention may contain one or more asymmetric carbon atoms and may exist in racemic and optically active form. All of these compounds and diastereoisomers are contemplated to be within the scope of the present invention.

Preferred compounds are those wherein:
P is CO₂R₆; more preferably P is CO₂H.
R₁ is hydrogen.
R₂ is Ar, cyclohexyl or C₁₋₄alkyl. More preferably R₂ is a group Ar wherein Ar is a group (a) or (b). In said group (a) or (b) Z₁ and Z₂ are independently hydrogen, CO₂R₆, (CH₂)ₙOH, C₁₋₄alkyl or C₁₋₆ alkoxy, e.g. methoxy; A is preferably CH₂, both Bs are preferably O.
R₃ and R₅ are independently hydrogen, CO₂R₆, OH, C₁₋₈alkoxy, C₁₋₈alkyl, N(R₆)₂, NO₂, Br, F, Cl, I, R₁₃CO₂R₇, X(CH₂)ₙR₈, (CH₂)ₘX'R₈, or X(C(R₆)₂)ₘOR₆;

In the context of the group R₃ and R₅ preferably do not represent hydrogen. In particular in the group R₃ preferably represents Br, Cl, C₁₋₈alkoxy e.g. methoxy; X(CH₂)ₙR₈, wherein X preferably represents O, n is 0, 1, or 2, and R₈ is preferably selected from:
CO₂R₆ wherein R₆ is preferably hydrogen;
OR₆ wherein R₆ is preferably H;
tetrazolyl optionally substituted by C₁₋₈alkyl e.g. ethyl;
CONR₇SO₂R₁₁ wherein R₇ is hydrogen or C₁₋₈alkyl e.g. methyl, R₁₁ preferably is C₁₋₈alkyl (e.g. methyl, isopropyl, or t-butyl) or phenyl optionally substituted by Br, Cl, F, C₁₋₈alkyl e.g. methyl;
or R₈ is phenyl or pyridyl substituted by one or more Br, Cl, CO₂H, CH₂OH.
R₅ is C₁₋₈alkoxy e.g. methoxy, or N(R₆)₂ wherein R₆ preferably is hydrogen or methyl.
R₄ is hydrogen, OH, C₁₋₅alkoxy, N(R₆)₂, Br, F, Cl, I, NHCOCH₃, or S(O)q C₁₋ ₅alkyl wherein the C₁₋₅alkyl may be unsubstituted or substituted by OH, methoxy or halogen. R₄ is more preferably hydrogen;
R₆ is hydrogen or C₁₋₈alkyl e.g. methyl and ethyl;
R₇ is hydrogen, C₁₋₁₀alkyl, C₂₋₁₀alkenyl or C₂₋₈alkynyl, all of which may be unsubstituted or substituted by one or more OH, N(R₆)₂, CO₂R₁₂, halogen, or R₇ is (CH₂)ₙAr. When R₇ is (CH₂)ₙAr, n is preferably zero or 1 and Ar is preferably phenyl unsubstituted or substituted by halogen or C₁₋₅ alkoxy.
R₁₁ is hydrogen, phenyl, or pyridyl wherein the phenyl and pyridyl may be unsubstituted or substituted by one or two C₁₋₄alkyl groups; C₁₋₈alkyl, C₂₋₈alkenyl, C₂₋₈alkynyl, all of which may be unsubstituted or substituted by one or more OH, CH₂OH, N(R₆)₂, or halogen;
R₁₂ is hydrogen or C₁₋₆alkyl.
R₁₃ is phenylene, pyridylene, or C₂₋₁₀alkylene, all of which may be unsubstituted or substituted by one or more CO₂R₆, OH, CH₂OH, N(R₆)₂, or halogen;
R₁₅ is preferably hydrogen or C₁₋₆alkyl e.g. ethyl, isopropyl, n-butyl, cyclopropylmethyl or cyclopropylethyl.
(Z) is preferably (d).

Preferred compounds are:
(E)-alpha-[[1-Butyl-5-[2-[(2-carboxyphenyl)methoxy]-4-methoxyphenyl]-1H-pyrazol-4-yl]methylene]-6-methoxy-1,3-benzodioxole-5-propanoic acid;
(E)-alpha-[[5-[2-[(2-Carboxyphenyl)methoxy]-4-methoxyphenyl)-1-ethyl-1H-pyrazol-4-yl]methylene]-6-methoxy-1,3-benzodioxole-5-propanoic acid;
(E)-alpha-[[1-Allyl-5-[2-[(2-carboxyphenyl)methoxy]-4-methoxyphenyl]-1H-pyrazol-4-yl]methylene]-6-methoxy-1,3-benzodioxole-5-propanoic acid;
(E)-alpha-[[1-Butyl-5-[2-[(2-carboxyphenyl)methoxy]-4-chlorophenyl]-1H-pyrazol-4-yl]methylene]-6-methoxy-1,3-benzodioxole-5-propanoic acid;
(E)-alpha-[[1-Butyl-5-[4-methoxy-2-[[N-(phenylsulfonyl)]carboxamidomethoxy]phenyl]-1H-pyrazol-4-yl]methylene]-6-methoxy-1,3-benzodioxole-5-propanoic acid;
(E)-3-[1-Butyl-5-[2-[(2-carboxyphenyl)methoxy]-4-methoxyphenyl]-1H-pyrazol-4-yl]-2-[(2,5-dimethoxyphenyl)methyl]-prop-2-enoic acid;
(E)-alpha-[[1-Butyl-5-[2-[(2-hydroxymethylphenyl)methoxy]-4-methoxyphenyl]-1H-pyrazol-4-yl]methylene]-6-methoxy-1,3-benzodioxole-5-propanoic acid;
(E)-alpha-[[1-Butyl-5-[2-[(2-carboxyphenyl)methoxy]-4-methoxyphenyl]-1H-pyrazol-4-yl]methylene]-7-methoxy-1,4-benzodioxan-6-propanoic acid;
(E)-alpha-[[5-[2-[(2-Carboxyphenyl)methoxy]-4-methoxyphenyl]-1-cyclopropylmethyl-1H-pyrazol-4-yl]methylene]-6-methoxy-1,3-benzodioxole-5-propanoic acid;
(E)-alpha-[[1-(3-Butenyl)-5-[2-[(2-hydroxymethylphenyl)methoxy]-4-methoxyphenyl]-1H-pyrazol-4-yl]methylene]-6-methoxy-1,3-benzodioxole-5-propanoic acid;
(E)-alpha-[[5-[2-[(2-Carboxyphenyl)methoxy]-4-methoxyphenyl]-1-cyclopropylethyl- 1H-pyrazol-4-yl]methylene]-6-methoxy-1,3-benzodioxole-5-propanoic acid;
(E)-alpha-[[1-Butyl-5-[2-[(2-carboxyphenoxy)methyl)4-methoxyphenyl]-1H-pyrazol-4-yl]methylene]-6-methoxy-1,3-benzodioxole-5-propanoic acid.

The present invention provides compounds of Formula (I), which may be made by methods similar to those given below.

Compounds of the Formula (Id): 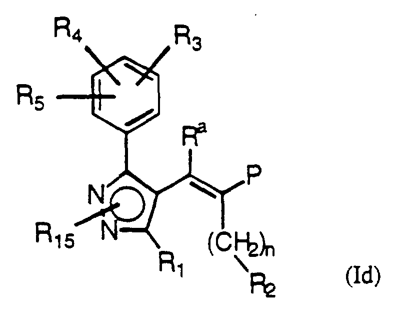 can be prepared by alkylating a ketone of Formula (2): 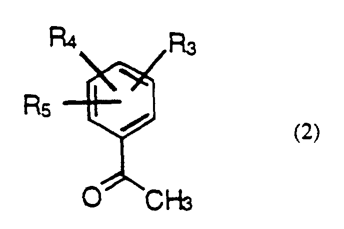 in dimethyl carbonate in the presence of sodium hydride to provide a β-keto ester of Formula (3).

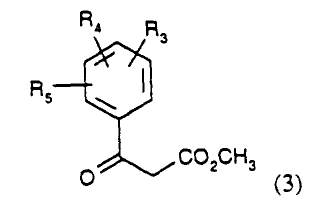

Condensation of a β-keto ester of Formula (3) with dimethyl formate dimethyl acetal in a suitable solvent such as toluene at approximately 95 °C affords a compound of Formula (4).

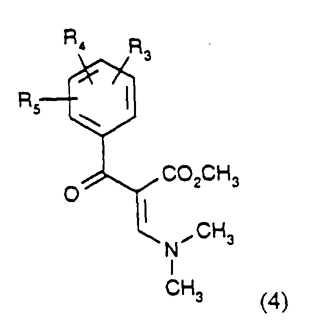

Treatment of a compound of Formula (4) with a hydrazine derivative of the Formula (5): 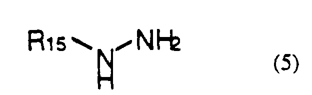 wherein R₁₅ is C₁₋₆alkyl;
in suitable solvents such as methanol and water in the presence of sodium acetate provides a pyrazole of Formula (6).

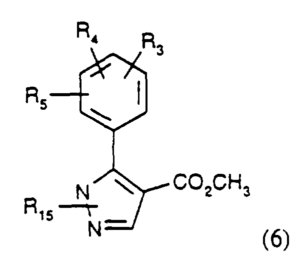

Reduction of an ester of Formula (6) with a reducing agent such as diisobutylaluminium hydride in a solvent such as dichloromethane followed by oxidation with an oxidant such as Jones reagent in acetone affords an aldehyde of Formula (7).

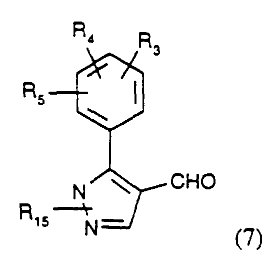

Knoevenagel condensation of an aldehyde of Formula (7) with a half acid of Formula (8), wherein R₁₆ is C₁₋₈ alkyl 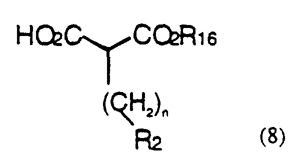 in a solvent such as benzene at reflux, in the presence of piperidinium acetate with azeotropic removal of water using a Dean-Stark apparatus affords an ester of Formula (9).

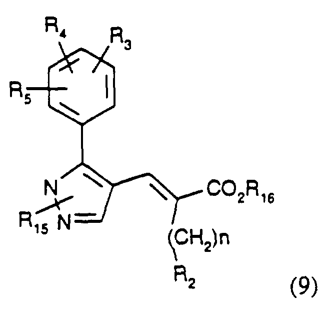

Followed if necessary and desired by:
1) deprotection and'alkylation and hydrolysis of the R₃, R₄, R₅, R₁₅, R₁₆, Z₁ and Z₂ groups as required and;
2) salt formation.
Aldehyde condensation may also be effectuated by heating in the presence of pyridine and acetic acid.

Conversion of an ester of formula (9) into an acid may be carried out using conventional deprotection techniques and hydrolysis.

A half acid of Formula (8): 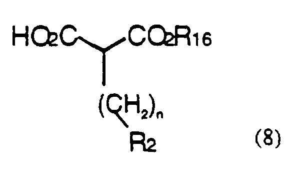 Wherein n=1, R₂ is (b), A=CH₂, B=O and R₁₆ is C₁₋₆ alkyl, may be prepared by reacting a compound of Formula (10) ; 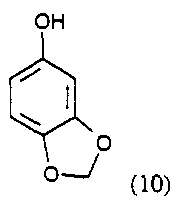 with methyl iodide in the presence of sodium hydride in a solvent such as dimethyl formamide to provide a compound of Formula (11).

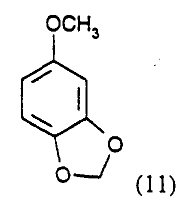

Treament of a compound of Formula (11) with POCl₃ in dimethyl formamide affords an aldehyde of Formula (12).

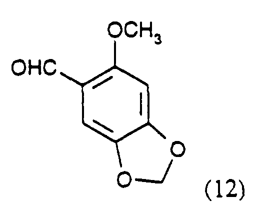

Condensation of an aldehyde of Formula (12) with a dialkyl malonate of Formula (13): 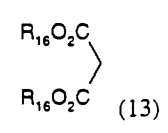 in the presence of piperidine and acetic acid in benzene provides a α,β-unsaturated ester of Formula (14).

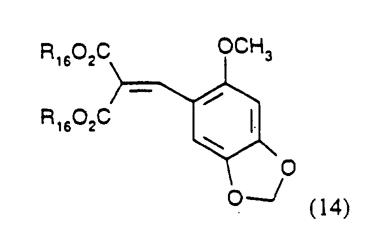

Reduction of a α,β-unsaturated ester of Formula (14) with sodium borohydride in a solvent such as ethanol gives a compound of Formula of (15).

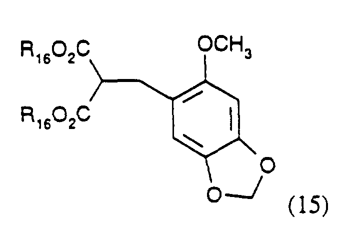

Monosaponification of a diester of Formula (15) with a base such as potassium hydroxide in a mixture of ethanol and water followed by acidic work up provides a half acid of Formula (8).
Other compounds of formula (Id) may be prepared by methods well known in the art. The invention also is a process for preparing compounds of Formula (Id) by:
(a) Reaction of a compound of Formula (II) 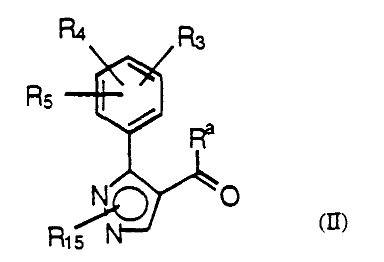 or a protected form or precursor thereof (as defined hereinafter) with a compound of Formula (8) 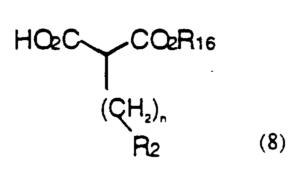 wherein R₂ and R₁₆ are as defined for Formula (Id) hereinabove;
   followed if necessary or desired by:
(b) conversion of one compound of Formula (Id) into a different compound of Formula (Id) e.g.
   (i) when Formula (Id) contains a group CO₂R₆, CO₂R₇ or CO₂R₁₂ wherein R₆, R₇ or R₁₂ is alkyl, conversion to a corresponding compound where R₆, R₇ or R₁₂ represents hydrogen;
   (ii) when Formula (Id) contains a hydroxy group (e.g. in R₃, R₄ or R₅) conversion to a different group, e.g. a group (CH₂)Ar where Ar is optionally substituted phenyl, by method well known in the art; and/or
(c) salt formation.

It will be appreciated by those skilled in the art that the substitutents R₃, R₄, R₅, R₁₅ and Z₁ and Z₂ may be introduced at any appropriate stage of the synthesis, preferably at an early stage, using methods well known in the art. In some of the reactions depicted above, particularly those in the early stages of the overall synthesis, one or more of the substitutents may therefore represent a precursor for the eventual substituent. A precursor for any of the substitutents means a group which may be derivatised or converted into the desired group. It will be further appreciated that it may be necessary or desirable to protect certain of these substitutents(or their precursors) at various stages in the reaction sequence. Suitable precursors and protecting groups are well known to those skilled in the art, as are methods for their conversion or removal respectively.

In another aspect the invention provides for an intermediate of the formula (II) wherein R₁₅, R₃, R₄, R₅ and R^{a} are as described for Formula (I)

Compounds of Formula (Ii) 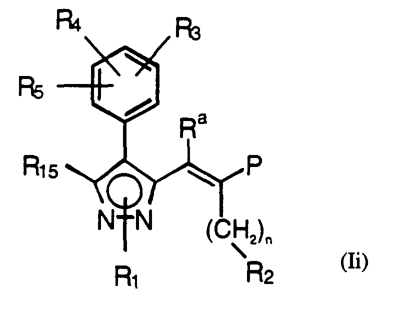 can be prepared starting by commercially available ketones of Formula (17): 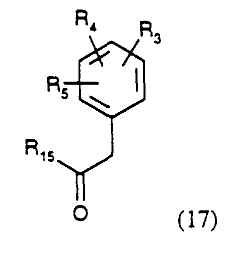 by reaction with diethyl oxalate of Formula (18): 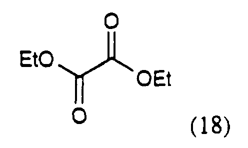 in the presence of a base such as sodium ethoxide in a solvent such as ethanol to produce a diketone of Formula (19).

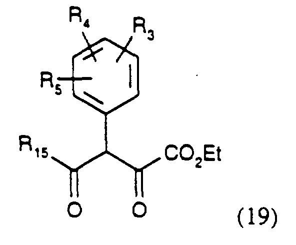

Reaction of a diketone of Formula (19) with hydrazine derivative of Formula (20): 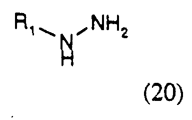 in a suitable solvent such as ethanol at reflux provides a pyrazole of Formula (21).

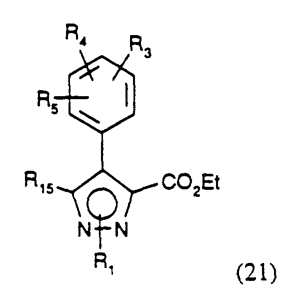

Saponification of an ester of Formula (21) using lithium hydroxide in a solvent such as aqueous methanol affords, after acidification an acid of the Formula (22): 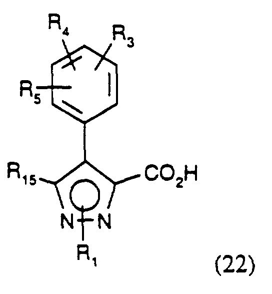 which can be subsequently converted to the corresponding N-methoxy-N-methylamide of Formula (23). 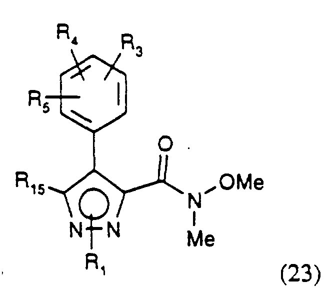 by treatment with methyl chloroformate followed by N,O-dimethylhydroxylamine hydrochloride in the presence of a base such as N-methylpiperidine. Compounds of Formula (23) can be treated with an organometallic reagent Ra-M wherein Ra is C₁₋₆ alkyl and m is Li or MgCl; to provide a compound of Formula (24), wherein R^{a} is C₁₋₆alkyl.

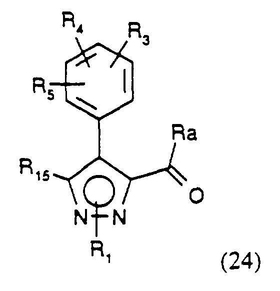

Reaction of compound of Formula (24) with the the lithium enolate of an ester of Formula (25): 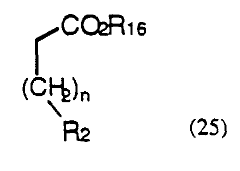 provides an alcohol of Formula (26).

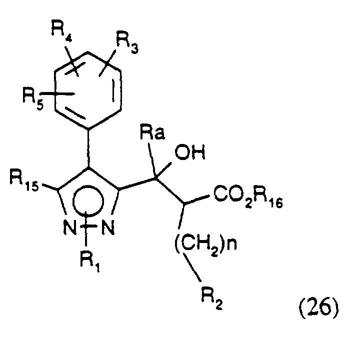

Dehydration of compound of Formula (26) with acetic anhydride followed by treatment with a base such as 1,8-diazabicyclo[5.4.0]undec-7-ene provides a compound of Formula (27).

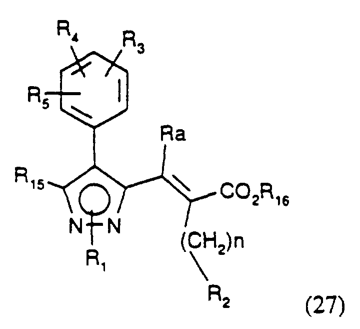

Alternatively, reaction of compound (24), wherein R^{a} is C₁₋₆alkyl, with Lawesson's reagent in a suitable solvent such as tetrahydrofuran affords a thione of Formula (28): 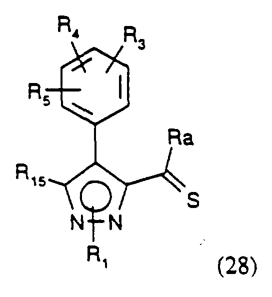 which can be treated with diazoester (29) : 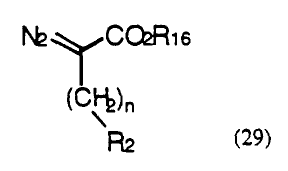 in refluxing tetrahydrofuran to provide a thiirane of Formula (30).

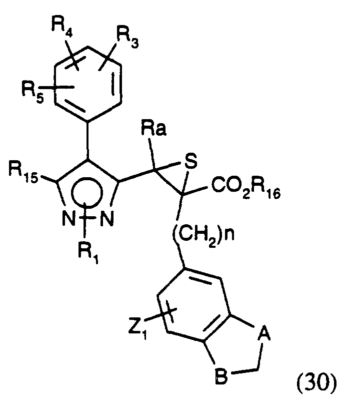

Treatment of a thiirane of Formula (30) with trimethylphosphite at reflux in a solvent such as chloroform provides compounds of Formula (27), wherein R^{a} is C₁₋₆alkyl.

Saponification of an ester of Formula (27) using lithium hydroxide in a solvent such as aqueous methanol affords, after acidification with acetic acid, an acid of the Formula (Ii), wherein P is CO₂H.

Compounds of the Formula (Ie) 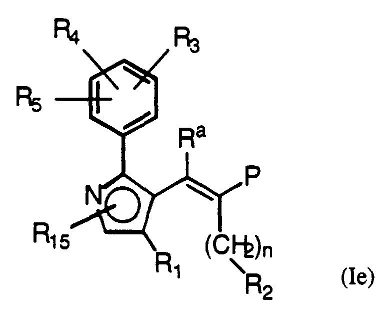 can be prepared following the steps outlined in the following Scheme 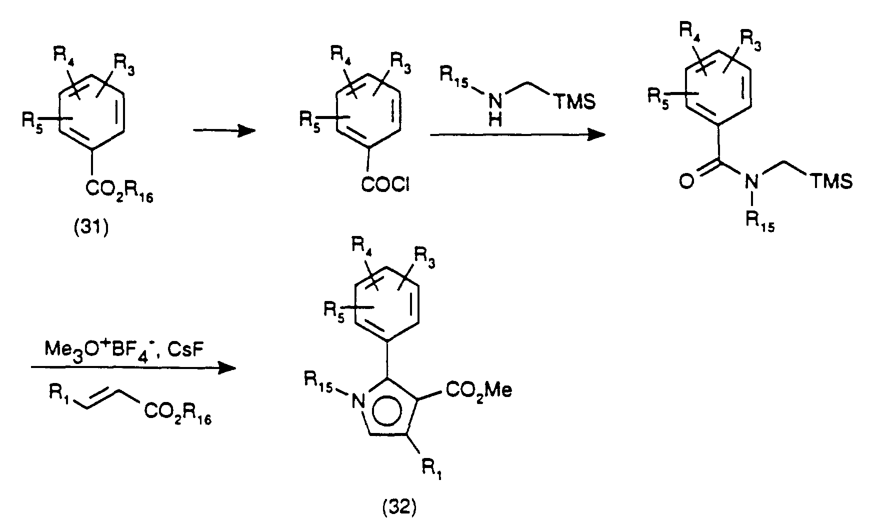 starting from an aryl ester of Formula (31), wherein R₁₆ is C₁₋₈ alkyl, to provide a pyrrole of Formula (32). Compound of Formula (32) can be subsequently converted to compounds of Formula (Ie) following the same sequence of steps as the one described above for the conversions of compound (6) and compound (21) to compounds (Id) and compound (Ii), respectively.

Compounds of Formula (Ih) may be prepared starting from a boronic acid of Formula (33): 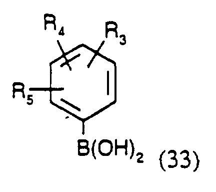 with a triazole of Formula (34), wherein X is Cr or Br 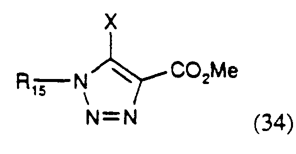 under standard Suzuki coupling conditions to provide an ester of Formula (35) .

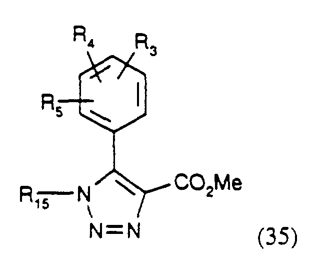

A compound of Formula (33) may be prepared by reaction of a corresponding organometallic derivative (eg. lithium or Grignard) with a trialkyl borate followed by hydrolysis.

A compound of Formula (34) may be prepared starting from dimethyl malonate with p-acetaminobenzenesulfonyl azide in a solvent such as acetonitrile in the presence of a base such as triethyl amine to provide dimethyl diazomalonate (36).

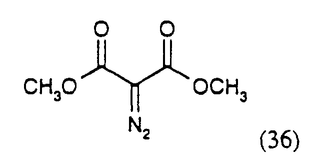

Treatment of diazomalonate of Formula (36) with an amine of Formula (37) : 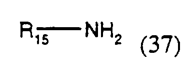 followed by acidic work up provides a triazole of Formula (38)

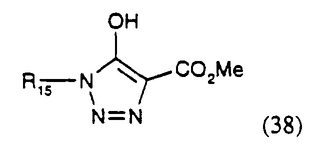

Reaction of a compound of Formula (38) with PX₅, wherein X is Br or Cl, in the presence of potassium carbonate in dimethylformamide affords a compound of Formula (34).

Compounds of Formula (Ij) may be prepared starting from an aniline of Formula (39) : 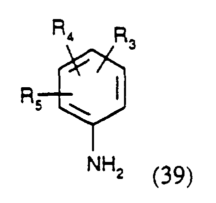 with a diketone of Formula of (40): 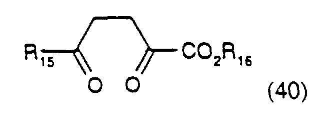 in a suitable solvent such as ethyl alcohol at reflux to provide a pyrrole of Formula (41).

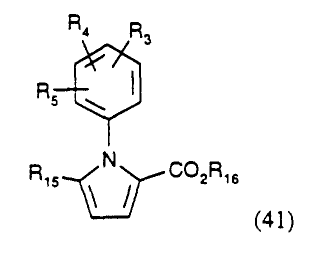

A diketone of Formula of (40) might be prepared by reacting of α,β-unsaturated ketone of Formula (42): 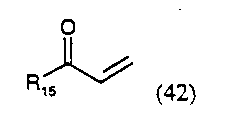 with a silyl enol ether of Formula (43): 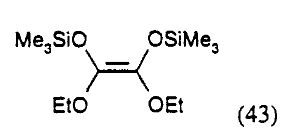 in the presence of Lewis acid such as zinc chloride in a suitable solvent such as dichloromethane followed by acidic hydrolysis.

Compounds of Formula (35) and compounds of Formula (41) can be subsequently converted to compounds of Formula (Ih) and compounds of Formula (Ij), respectively, following the same sequence of steps as the one described above for the conversions of compound (6), compound (21) and compound (32) to compounds (Id), compound (Ii) and compound (Ie), respectively.

In order to use a compound of the Formula (I) or a pharmaceutically acceptable salt thereof for the treatment of humans and other mammals it is normally formulated in accordance with standard pharmaceutical practice as a pharmaceutical composition.

Compounds of Formula (I) and their pharmaceutically acceptable salts may be administered in a standard manner for the treatment of the indicated diseases, for example orally, parenterally, sub-lingually, transdermally, rectally, via inhalation or via buccal administration.

Compounds of Formula (I) and their pharmaceutically acceptable salts which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, agar, pectin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile aqueous or non-aqueous carrier optionally containing a parenterally acceptable oil, for example polyethylene glycol, polyvinylpyrrolidone, lecithin, arachis oil, or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of Formula (1) or a pharmaceutically acceptable salt thereof which is active when administered in this way, with a binding and/or lubricating agent, for example polymeric glycols, gelatins, cocoa-butter or other low melting vegetable waxes or fats or their synthetic analogues.

Typical transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer to themselves a single dose.

Each dosage unit for oral administration contains suitably from 0.1 mg to 500 mg/Kg, and preferably from 1 mg to 100 mg/Kg, and each dosage unit for parenteral administration contains suitably from 0.1 mg to 100 mg, of a compound of Formula (I) or a pharmaceutically acceptable salt thereof calculated as the free acid. Each dosage unit for intranasal administration contains suitably 1-400 mg and preferably 10 to 200 mg per person. A topical formulation contains suitably 0.01 to 1.0% of a compound of Formula (I).

The daily dosage regimen for oral administration is suitably about 0.01 mg/Kg to 40 mg/Kg, of a compound of Formula (I) or a pharmaceutically acceptable salt thereof calculated as the free acid. The daily dosage regimen for parenteral administration is suitably about 0.001 mg/Kg to 40 mg/Kg, of a compound of the Formula (I) or a pharmaceutically acceptable salt thereof calculated as the free acid. The daily dosage regimen for intranasal administration and oral inhalation is suitably about 10 to about 500 mg/person. The active ingredient may be administered from 1 to 6 times a day, sufficient to exhibit the desired activity.

No unacceptable toxicological effects are expected when compounds of the invention are administered in accordance with the present invention.

The biological activity of the compounds of Formula (I) are demonstrated by the following tests:

### I. Binding Assay

### A) CHO cell membrane preparation.

CHO cells stably transfected with human ET_{A} and ET_{B} receptors were grown in 245 mm x 245 mm tissue culture plates in Dulbecco's modified Eagle's medium supplemented with 10% fetal bovine serum. The confluent cells were washed with Dulbecco's phosphate-buffered saline containing a protease inhibitor cocktail (5 mM EDTA, 0.5 mM PMSF, 5 µg/ml of leupeptin and 0.1 µg/ml of aprotinin) and scraped in the same buffer. After centrifugation at 800 *x g*, the cells were lysed by freezing in liquid nitrogen and thawing on ice followed by homogenization (30 times using a glass dounce homogenizer) in lysis buffer containing 20 mM Tris HCI, pH 7.5, and the protease inhibitor cocktail. After an initial centrifugation at 800 x *g* for 10 min to remove unbroken cells and nuclei, the supernatants were centrifuged at 40,000 *x g* for 15 min and the pellet was resuspended in 50 mM Tris HCI, pH 7.5, and 10 mM MgCl₂ and stored in small aliquots at -70°C after freezing in liquid N₂. Protein was determined by using the BCA method and BSA as the standard.

### (B) Binding studies.

[¹²⁵I]ET-1 binding to membranes prepared from CHO cells was performed following the procedure of Elshourbagy *et al.* (1993). Briefly, the assay was initiated in a 100 µl volume by adding 25 µl of [¹²⁵I]ET-1 (0.2-0.3 nM) in 0.05% BSA to membranes in the absence (total binding) or presence (nonspecific binding) of 100 nM unlabeled ET-1. The concentrations of membrane proteins were 0.5 and 0.05 µg per assay tube for ET_{A} and ET_{B} receptors, respectively. The incubations (30°C, 60 min) were stopped by dilution with cold buffer (20 mM Tris HCI, pH 7.6, and 10 mM MgCl₂) and filtering through Whatman GF/C filters (Clifton, NJ) presoaked in 0.1% BSA. The filters were washed 3 times (5 ml each time) with the same buffer by using a Brandel cell harvester and were counted by using a gamma counter at 75% efficiency.

The following examples are illustrative and are not limiting of the compounds of this invention.

### EXAMPLE 1

### (E)-alpha-[[1-n-Butyl-5-[2-[(2-carboxyphenyl)methoxy]-4-methoxyphenyl]-1H-pyrazol-4-yl]methylene]-6-methoxy-1,3-benzodioxole-5-propanoic acid

### a) (E)-Ethyl 6-Methoxy-alpha-[(7-methoxy-4-oxo-4H-1-benzopyran-3-yl)methylene]-1,3-benzodioxole-5-propanoate

A solution of 3-formyl-7-methoxychromone (0.67 g, 3.3 mmol) and ethyl hydrogen 2-[(6-methoxy-3,4-methylenedioxy)benzyl]malonate (0.89 g, 3.0 mmol) in benzene (30 mL) was treated with piperidine (0.15 mL, 1.5 mmol) followed by acetic acid (0.085 mL, 1.5 mmol). The reaction was stirred at reflux equipped with a Dean Stark apparatus for 2 h. The mixture was cooled then extracted with EtOAc (200 mL). The organic extract was washed successively with saturated NaHCO₃ and brine, dried (MgSO₄) and concentrated under vacuum. The resulting residue was purified by column chromatography (silica gel, EtOAc/hexane, gradient 75:25 to 70:30) to afford a material consisting of a 1.2 :1 mixture of E:Z enoates as an oil (1.02 g, 78%). Recrystallization of this material from ethanol affords the title compound as the E-isomer, exclusively.
Data for the the E-isomer: mp 140-141°C; MS (ESI) *m*/*z* 439 (M+H)⁺. Anal.
Calcd for C₂₄H₂₂O₈: C, 65.75; H, 5.06. Found: C, 65.56; H, 4.99.

### b) (E)-Ethyl alpha-[[1-Butyl-5-(2-hydroxy-4-methoxyphenyl)-1H-pyrazol-4-yl]methylene]-6-methoxy-1,3-benzodioxole-5-propanoate and (E)-Ethyl alpha-[[1-Butyl-3-(2-hydroxy-4-methoxyphenyl)-1H-pyrazol-4-yl]methylene]-6-methoxy-1,3-benzodioxole-5-propanoate

A solution of the compound of Example 1(a) (0.679 g, 1.55 mmol), butylhydrazine oxalate (0.552 g, 3.1 mmol) and sodium acetate trihydrate (1.265 g, 9.3 mmol) in a mixture of 9:1 EtOH:H₂O (31 mL) was stirred at reflux for 1 h. The reaction mixture was cooled and subsequently partitioned between EtOAc (300 mL) and aqueous pH 7 buffer. The organic extract was washed with brine, dried (Na₂SO₄) and concentrated under vacuum. The resulting residue was purified by column chromatography (silica gel, EtOAc/hexane, 60:40) to afford the 1,3,4-pyrazole (340 mg, 43%) followed by the isomeric 1,4,5-pyrazole (361 mg, 46%). Data for the 1,4,5-pyrazole: *Rf* (70:30 hexane:ethyl acetate) 0.14; ¹H NMR (400 MHz, CDCl₃) δ 0.79 (t, *J* = 7.3 Hz, 3 H), 1.12-1.21 (m, 2 H), 1.20 (t, *J* = 7.2 Hz, 3 H), 1.67 (quintet, *J* = 7.4 Hz, 2 H), 3.79 (s, 3 H), 3.83 (s, 3 H), 3.86-3.95 (m, 4 H), 4.14 (q, *J* = 7.2 Hz, 2 H), 5.84 (s, 2 H), 6.53 (s, 1 H), 6.54 (s, 1 H), 6.56 (d, *J* = 2.4 Hz, 1 H), 6.61 (dd, *J* = 8.5, 2.4 Hz, 1 H), 7.03 (d, *J* = 8.5 Hz, 1 H), 7.39 (s, 1 H), 7.60 (s, 1 H); MS (ESI) *m*/*z* 509 (M+H)⁺.
Data for 1,3,4-pyrazole: *Rf* (70:30 hexane:ethyl acetate) 0.41; ¹H NMR (400 MHz, CDCl₃) δ 0.91 (t, *J* = 7.3 Hz, 3 H), 1.22-1.25 (m, 2 H), 1.28 (t, *J* = 7.2 Hz, 3 H), 1.80 (quintet, *J* = 7.4 Hz, 2 H), 3.76-3.81 (m, 2 H), 3.77 (s, 3 H), 3.78 (s, 2 H), 4.04 (t, *J* = 7.1 Hz, 2 H), 4.24 (q, *J* = 7.2 Hz, 2 H), 5.88 (s, 2 H), 6.49-6.54 (m, 1 H), 6.55 (s, 2 H), 6.61 (d, J = 2.5 Hz, 1 H), 7.32 (s, 1 H), 7.42 (d, J = 8.5 Hz, 1 H), 7.92 (s, 1 H), 10.5 (s, 1 H); MS (ESI) *m*/*z* 509 (M+H)⁺.

### c) (E)-Ethyl alpha-[[1-Butyl-5-[4-methoxy-2-[[2-(methoxycarbonyl)phenyl]methoxy]phenyl]-1H-pyrazol-4-yl]methylene]-6-methoxy-1,3-benzodioxole-5-propanoate

A solution of the 1,4,5-pyrazole of Example 1(b) (0.34 g, 0.67 mmol) in DMF (2.0 mL) was added dropwise to a slurry of NaH (0.024 g, 1.0 mmol) in DMF (1.3 mL) at room temperature. The reaction was stirred for 3 min at which time was added methyl 2-(bromomethyl)benzoate (0.23 g, 1.0 mmol) and stirring continued for 1 h at room temperature. The mixture was quenched with aqueous pH 7 buffer, then diluted with EtOAc. The organic extract was washed with brine, dried (Na₂SO₄) and concentrated under vacuum. The resulting residue was purified by column chromatography (silica gel, gradient hexane/Et₂O, 60:40 to 50:50) to afford the title compound (396 mg, 90%). *Rf* (70:30 hexane:ethyl acetate) 0.23 ; MS (ESI) *m*/*z* 657 (M+H)⁺.

### d) (E)-alpha-[[1-Butyl-5-[2-[(2-carboxyphenyl)methoxy]-4-methoxyphenyl]-1H-pyrazol-4-yl]methylene]-6-methoxy-1,3-benzodioxole-5-propanoic Acid

To a solution of the ester of Example 1(c) (0.396 g, 0.60 mmol) in MeOH (16.2 mL) was added aqueous LiOH•H₂O (0.253 g, 6.0 mmol, in 1.8 mL H₂O), and the reaction was heated at reflux for 4 h. After cooling to room temperature, acetic acid (2.5 mL) was added followed by H₂O (15 mL) at which time a precipitate formed. Most of the methanol was removed under reduced pressure, and the resulting aqueous solution was cooled to 0°C. The mixture was filtered and the precipitate was recrystallized from EtOAc (-20 °C) to give the title compound as white crystals (0.27 g). The mother liquors were purified by column chromatography (silica gel, gradient CH₂Cl₂/hexane/EtOAC/HOAc 40:40:20:1 to 40:30:30:1) to afford an additional 86 mg of title compound (356 mg, 96% overall): mp 213-214°C; *Rf* (50:50:1 hexane:ethyl acetate: acetic acid) 0.43; ¹H NMR (400 MHz, CDCl₃) δ 0.80 (t, *J* = 7.3 Hz, 3 H), 1.21-1.26 (m, 2 H), 1.69-1.77 (m, 2 H), 3.59 (s, 2 H), 3.81 (s, 3 H), 3.86 (s, 3 H), 3.97-4.02 (m, 2 H), 5.53 (s, 2 H), 5.82 (s, 2 H), 6.47 (s, 1 H), 6.52 (s, 1 H), 6.67 (dd, *J* = 8.4, 2.2 Hz, 1 H), 6.70 (d, *J* = 2.2 Hz, 1 H), 7.12 (d *J* = 8.4 Hz, 1 H), 7.32-7.36 (m, 2 H), 7.48 (t, *J* = 7.6 Hz, 1 H), 7.52 (s, 1 H), 7.54 (s, 1 H), 8.08 (d, *J* = 7.4 Hz, 1 H); MS (ESI) *m/z* 613 (M-H)⁻. Anal. Calcd for C₃₄H₃₄N₂O₉ • 0.25 H₂O: C, 65.96; H, 5.62; N, 4.52. Found: C, 65.94; H, 5.44; N, 4.44.

Alternately 1-butyl-5-[2-(2-carboxyphenyl)methoxy]-4-methoxyphenylpyrazole-4-carboxyaldehyde may be prepared as follows.

### a) (E)-Methyl 2-[[2-(3-hydroxy-1-oxo-2-propenyl)-5-methoxyphenoxy]methyl]benzoate

To a 12 L round bottomed flask, was added 60% sodium hydride (50.9 g, 1.27 mol, 2 equivalents). Toluene (600 mL) was added and the resulting solution was stirred. Methyl 2-[(2-acetyl-5-methoxyphenoxy)methyl]benzoate (200.0 g, 0.636 mol) dissolved in toluene (2.0 L) was added slowly over a period of 20 minutes. After the addition was complete, the solution was stirred for a period of 15 minutes. Methyl formate (229.1 g, 235 mL, 3.82 mol) was added over 20 minutes. The mixture was stirred for 1 hour at ambient temperature. Sodium methoxide (34.3 g, 0.636 mol) was added in three equal portions over 30 minutes. The mixture was stirred at ambient for 16 hours. HPLC of the reaction mixture showed the absence of starting material. Hexane (6 L) was added and the reaction mixture was stirred for 1 hour. The solids were filtered and washed with 2 L of hexane and dried under vacuum (0.5 mTorr) to afford (E)-methyl 2-[[2-(3-hydroxy-1-oxo-2-propenyl)-5-methoxyphenoxy]methyl]benzoate, monosodium salt (303.9 g). The (E)-methyl 2-[[2-(3-hydroxy-1-oxo-2-propenyl)-5-methoxyphenoxy]methyl]benzoate, monosodium salt was slurried in water (3 L) and toluene (4 L) was added. A solution of 10% citric acid (2.5 L) was added and the solution stirred for 1 hour. The layers were separated, and the aqueous layer was extracted with 2 L of toluene. The combined toluene layers were washed with brine solution (2 x 2 L) and dried (magnesium sulfate), filtered and concentrated to a solid which was triturated with hexane (2 L). The solid was filtered and dried to afford the title compound (188.4 g, 86.6% yield) as a light yellow solid: ¹H NMR (300 MHz, CDCl₃) δ 8.20 (1H, d), 8.05 (1H, d), 7.95 (1H, m), 7.70 (1H, d), 7.65 (1H, m), 7.45 (1H, m), 6.50 (2H, m), 5.65 (2H, m), 5.3 (1H, br s), 3.90 (3H, s), 3.80 (3H, s).

### b) (E)-Methyl 2-[[2-[3-(diethylamino)-1-oxo-2-propenyl]-5-methoxyphenoxy]methyl]benzoate

To a 2 L round bottomed flask was added (E)-methyl 2-[[2-(3-hydroxy-1-oxo-2-propenyl)-5-methoxyphenoxy]methyl]benzoate (104 g, 0.304 mol) and 1 L of toluene. After complete dissolution, diethylamine (28.3g, 40 mL, 0.39 mol) was rapidly added under nitrogen via an addition funnel into the stirred solution. The reaction mixture was stirred under nitrogen for 1 h and it was then concentrated under reduced pressure to give approximately 120 g (100% yield) of the title compound as an orange solid: ¹H NMR (300 MHz, CDCl₃) δ 8.02 (1H, d), 7.84(1H, d) 7.67 (2H, bd) 7.51 (1H, t) 7.35 (1H, t), 6.54 (2H, m), 5.75 (1H, d), 5.52 (2H, s), 3.80 (3H, s), 3.23 (4h, br), 1.14 (6H, br).

### c) 1-Butyl-5-[2-(2-methoxycarbonylphenylmethoxy)-4-methoxyphenyl-1H-pyrazole

To a 5 L round bottomed flask was added (E)-methyl 2-[[2-[3-(diethylamino)-1-oxo-2-propenyl]-5-methoxyphenoxy]methyl]benzoate (150 g, 0.37 mol), 2.9 L of methanol and 0.45 L of water. After complete dissolution, a premixed quantity of sodium acetate (294 g, 3.58 mol) and n-butylhydrazine oxalate salt (133 g, 0.746 mol) was added via a powder funnel in rapid portions over a period of 20 minutes. The reaction mixture was blanketed with nitrogen during the reaction period. After 20 hours the reaction mixture was partitioned between 3 L of ethyl acetate and 3 L of water. The aqueous was removed and poured into 2 L of ethyl acetate and the aqueous was separated once again. This operation was repeated once more with 1.2 L of ethyl acetate (a total of 6 L of ethyl acetate used in total). The combined organic extracts were dried (magnesium sulfate) and concentrated under reduced pressure to 187 g of a thick oil. Flash chromatography (50% TBME/Hex) afforded the title compound (153 g, 73% yield) as a colourless oil which slowly solidified on standing: ¹H NMR (300 MHz, CDCl₃) δ 8.02 (1H, d), 7.6 (1H, d), 7.52-7.22 (3H, m), 7.12 (1H, d), 6.64 (1H, d), 6.54 (1H, dd), 6.20 (1H, s), 5.48 (2H, s), 4.01 (2H, t), 3.89 (s 3H), 3.76 (3H, s), 1.7-1.5 (2H, m), 1.2-1.05 (2H, m), 0.7 (3H, t).

### d) 1-Butyl-5-[2-(2-methoxycarbonylphenylmethoxy)-4-methoxyphenyl-1H-pyrazole-4-carboxyaldehyde

To a 2 L round bottomed flask was added 235 mL of DMF, followed by phosphorous oxychloride (125.0 g, 75 mL, 0.81 mol) under nitrogen. The temperature of the reaction mixture rose to 40 °C during the addition. The mixture was then allowed to cool to room temperature and then was poured into a separate flask containing 235 mL of DMF and 1-butyl-5-[2-(2-methoxycarbonylphenylmethoxy)-4-methoxyphenyl-1H-pyrazole (145 g, 0.36 mol). The resulting mixture was warmed to 95 °C under nitrogen; it was then allowed to cool and was poured into 1 L of water containing sodium acetate (100g, 1.13 mol). TBME (1.5 L) was added and the organic phase was separated. An additional 1.5 L of TBME was added. The combined organic phases were dried with magnesium sulfate , filtered and concentrated to 200 g of crude product as a dark oil. Flash chromatography (30% TBME/hexane to 60% hex/TBME) afforded 100 g of solid which was recrystallized (TBME/ Hex) to give the title compound (91.3g, 58% yield) as a white solid: ¹H NMR (300 MHz, CDCl₃) δ 9.54 (1H, s), 8.1 (1H, s), 8.02 (1H, d), 7.45 (1H, t), 7.31 (1H, t), 7.29-7.261 (1H, m), 7.20 (1H, d), 6.71 (1H, s), 6.55 (1H, d), 5.5 (2H, s), 4.0 (2H, t), 3.92 (3H,s), 1.82-1.64 (2H, m), 1.22-1.08 (2H, m), 0.8 (3H, t).

### e) 1-Butyl-5-[2-(2-carboxyphenyl)methoxy]-4-methoxyphenylpyrazole-4-carboxyaldehyde

To a 2 L round bottomed flask equipped with an air driven stirrer and condenser was added 1-butyl-5-[2-(2-methoxycarbonylphenylmethoxy)-4-methoxyphenyl-1H-pyrazole-4-carboxyaldehyde (92 g, 0.218 mol), 0.883 L of methanol and lithium hydroxide (14.1 g, 0.336 moles, 1.54 eq). The mixture was warmed to 70 °C and 0.222 L of water was added and the resultant light yellow mixture was maintained at 65 °C for 2 hours under nitrogen atmosphere. The solution was allowed to cool and then was concentrated under reduced pressure to give a thick oil. The oil was sequentially treated with citric acid (60 g, 0.285 moles, 1.3 eq) predissolved in 1.0 L of water and 1 L of dichloromethane. The organic layer was removed and the aqueous layer was washed with 0.5 L of dichloromethane. The organic layers were combined, dried (magnesium sulfate), filtered through a pad of celite, and then concentrated under reduced pressure. This oil was redissolved in 0.8 L of toluene and placed in a -20 °C freezer for 12 hours. The solid formed was filtered, washed with 500 mL of toluene and placed in an oven and dried to constant weight (1.0 Torr @ 50°C) to afford the title compound (72 g, 81% yield) as a white solid: ¹H NMR (300 MHz, CDCl₃) δ 9.55 (1H, s), 8.15-8.10 (2H, m), 7.51 (1H, t), 7.35 (1H, t), 7.28 (1H, d), 7.20 (1H, d), 6.70 (1H, s), 6.52 (1H,dd), 5.51 (2H, s), 4.1-3.90 (2H, m), 3.85 (3H, s), 1.82-1.64 (2H, m), 1.22-1.08 (2H, m), 0.8 (3H, t).

### EXAMPLE 2

### (E)-alpha-[[1-n-Butyl-3-[2-[(2-carboxyphenyl)methoxy]-4-methoxyphenyl]-1H-pyrazol-4-yl]methylene]-6-methoxy-1,3-benzodioxole-5-propanoic acid

### a) (E)-Ethyl alpha-[[1-Butyl-3-[4-methoxy-2-[[2-(methoxycarbonyl)phenyl]methoxy]phenyl]-1H-pyrazol-4-yl]methylene]-6-methoxy-1,3-benzodioxole-5-propanoate

To a solution of the 1,3,4-pyrazole of Example 1(b) (0.23 g, 0.45 mmol) in DMF (5 mL) was added NaH (0.04 g, 1.83 mmol) at 0°C. The reaction stirred for 3 min at which time was added methyl 2-(bromomethyl)benzoate (0.22 g, 0.80 mmol) and stirring continued for 1.5 h at room temperature. The mixture was partitioned with EtOAc and 2% HCI, the resulting organic layer was then washed with water (2X). The aqueous layer was then neutralized with NaHCO₃ to pH 7 and then partitioned with EtOAc. The combined organic extract was washed with brine, dried (MgSO₄) and concentrated under vacuum. The resulting residue was purified by column chromatography (silica gel, gradient hexane/Et₂O, 67:33 to 65:35) to afford the title compound as an oil (0.25 g, 81%). ¹H NMR (400 MHz, CDCl₃) δ 0.91 (t, *J* = 7.4 Hz, 3 H), 1.13 (t, *J* = 7.1 Hz, 3 H), 1.30 (sextet, *J* = 7.4 Hz, 2 H), 1.83 (quintet, *J* = 7.4 Hz, 2 H), 3.73 (s, 2 H), 3.85 (s, 3 H), 3.86 (s, 3 H), 3.89 (s, 3 H), 4.07 (q, *J* = 7.1 Hz, 2 H), 4.08 (t, *J* = 7.4 Hz, 2 H), 5.51 (s, 2 H), 5.87 (s, 2 H), 6.49 (s, 1 H), 6.59 (s, 1 H), 6.61 (dd, *J* = 8.4, 2.2 Hz, 1 H), 6.69 (d, *J* = 2.2 Hz, 1 H), 7.31 (t, *J* = 7.8 Hz, 1 H), 7.34 (d, *J* = 8.4 Hz, 1 H), 7.39 (s, 1 H), 7.46 (t, *J* = 7.7 Hz, 1 H), 7.61 (d, *J* = 7.8 Hz, 1 H), 7.70 (s, 1 H), 7.99 (d, *J* = 7.8 Hz, 1 H).

### b) (E)-alpha-[[1-Butyl-3-[2-[(2-carboxyphenyl)methoxy]-4-methoxyphenyl]-1H-pyrazol-4-yl]methylene]-6-methoxy-1,3-benzodioxole-5-propanoic Acid

To a solution of the ester of example 2(a) (0.21 g, 0.33 mmol) in MeOH (13.5 mL) was added aqueous LiOH (0.13 g, 3.0 mmol, in 1.5 mL H₂O), and the reaction was heated at reflux for 5 h. To the mixture was added acetic acid (1 mL) and most of the solvent was removed under reduced pressure. The residue was partitioned between EtOAc and brine. The organic layer was dried (MgSO₄) and concentrated under vacuum. The resulting residue was purified by column chromatography (silica gel, CH₂Cl₂/EtOAc/hexane/HOAc, 40:30:30:1) to give a white solid, which was recrystallized from EtOAc to afford the title compound (0.175 g, 86%): mp 226-227 °C; ¹H NMR (400 MHz, CDCl₃) δ 0.94 (t, *J* = 7.3 Hz, 3 H), 1.33 (sextet, *J* = 7.3 Hz, 2 H), 1.85 (quintet, *J* = 7.3 Hz, 2 H), 3.44 (s, 2 H), 3.82 (s, 3 H), 3.85 (s, 3 H), 4.10 (t, *J* = 7.2 Hz, 2 H), 5.65 (s, 2 H), 5.85 (s, 2 H), 6.47 (s, 1 H), 6.57 (s, 1 H), 6.62 (dd, *J* = 8.4, 2.1 Hz, I H), 6.67 (d, *J* = 2.1 Hz, 1 H), 7.32-7.37 (m, 2 H), 7.43 (d *J* = 8.4 Hz, 1 H), 7.48-7.52 (m, 2 H), 7.76 (s, 1 H), 8.12 (d, *J* = 7.9 Hz, 1 H); MS (ESI) *m/z* 615 (M+H)⁺. Anal. Calcd for C₃₄H₃₄N₂O₉ • 0.25 H₂O: C, 65.97; H, 5.62; N, 4.52. Found: C, 65.84; H, 5.46; N, 4.31.

### EXAMPLE 3

### (E)-alpha-[[1-n-Butyl-5-[2-[(2-carboxyphenyl)methoxy]-5-chloro-4-methylphenyl]-1H-pyrazol-4-yl]methylene]-6-methoxy-1,3-benzodioxole-5-propanoic acid

### a) (E)-Ethyl 6-Methoxy-alpha-[(6-chloro-7-methyl-4-oxo-4H-1-benzopyran-3-yl)methylene]-1,3-benzodioxole-5-propanoate

Following the procedure of Example 1(a), except substituting 6-chloro-3-formyl-7-methylchromone for 3-formyl-7-methoxychromone, the title compound was prepared in 38% yield: mp (hexane/ethyl acetate) 134-135 °C.

### b) (E)-Ethyl alpha-[[1-Butyl-5-(5-chloro-2-hydroxy4-methylphenyl)-1H-pyrazol-4-yl]methylene]-6-methoxy-1,3-benzodioxole-5-propanoate

Following the procedure of Example 1(b), except substituting the compound of Example 3(a) for (*E*)-ethyl 6-methoxy-*alpha*-[(7-methoxy-4-oxo-4H-1-benzopyran-3-yl)methylene]-1,3-benzodioxole-5-propanoate, the title compound was prepared in 41% yield: MS (ESI) *m/z* 527 (M+H)⁺.

### c) (E)-Ethyl alpha-[[1-Butyl-5-[5-chloro-2-[[2-(methoxycarbonyl)phenyl]methoxy]-4-methylphenyl]-1H-pyrazol-4-yl]methylene]-6-methoxy-1,3-benzodioxole-5-propanoate

Following the procedure of Example 1(c), except substituting the compound of Example 3(b) for (*E*)-ethyl *alpha*-[[1-butyl-5-(2-hydroxy-4-methoxyphenyl)-1H-pyrazol-4-yl]methylene]-6-methoxy-1,3-benzodioxole-5-propenoate, the title compound was prepared in 90% yield: MS (ESI) *m/z* 675 (M+H)⁺.

### d) (E)-alpha-[[1-Butyl-5-[2-[(2-carboxyphenyl)methoxy]-5-chloro-4-methylphenyl]-1H-pyrazol-4-yl]methylene]-6-methoxy-1,3-benzodioxole-5-propanoic Acid

Following the procedure of Example 1(d), except substituting the compound of Example 3(c) for (*E*)-ethyl *alpha*-[[1-butyl-5-[4-methoxy-2-[[2-(methoxycarbonyl)phenyl]methoxy]phenyl]-1H-pyrazol-4-yl]methylene]-6-methoxy-1,3-benzodioxole-5-propanoate, the title compound was prepared in 83% yield. mp 114-116°C.

### EXAMPLE 4

### (E)-alpha-[[1-n-Butyl-5-[2-[(2-carboxyphenyl)methoxy]-4-chlorophenyl]-1H-pyrazol-4-yl]methylene]-6-methoxy-1,3-benzodioxole-5-propanoic acid 110°C (dec)

### EXAMPLE 5

### (Z)-alpha-[[1-n-Butyl-5-[2-[(2-carboxyphenyl)methoxyl-4-methoxyphenyl)-1H-pyrazol-4-yl]methylene]-6-methoxy-1,3-benzodioxole-5-propanoic acid 188-189°C

### EXAMPLE 6

### (E)-alpha-[[1-Hydro-(3-[(2-carboxyphenyl)methoxyl-4-methoxyphenyl]-1H-pyrazol-4-yl]methylene]-6-methoxy-1,3-benzodioxole-5-propionic acid

### EXAMPLE 7

### (E)-alpha-[[1-Ethyl[5-[(2-carboxyphenyl)methoxy]methoxyphenyl]-1H-pyrazol-4-yl]methylene]-6-methoxy-1,3-benzodioxole-5-propionic acid

### EXAMPLE 8

### (E)-alpha-[[1-Allyl-[5-(2-carboxyphenyl)methoxy]-4-methoxyphenyl]-1H-pyrazol-4-yl]methylene]-6-methoxy-1,3-benzodioxole-5-propionic acid

### EXAMPLE 9

### (E)-alpha-[[1-n-Butyl-5-[2-[(2-carboxyphenyl)methoxy]-4,5-dichlorophenyl]-1H-pyrazol-4-yl]methylene]-6-methoxy-1,3-benzodioxole-5-propanoic acid 226-228°C

### EXAMPLE 10

### (E)-alpha-[[1-n-Butyl-[3-[4-methoxy-2-[[N-(phenylsulfonyl)]carboxamidomethoxy]phenyl]-1H-pyrazol-4-yl]methylene]-6-methoxy-1,3-benzodioxole propionic acid

### EXAMPLE 11

### (E)-3-[1-n-Butyl-5-[2-[(2-carboxyphenyl)methoxy]-4-methoxyphenyl]-1H-pyrazol-4-yl]-2-[(3,5-dimethoxyphenyl)methyl]-prop-2-enoic acid

### EXAMPLE 12

### (E)-alpha-[[1-n-Butyl-5-[4-methoxy-2-[[N-(phenylsulfonyl)lcarboxamidomethoxy]phenyl]-1H-pyrazol-4-yl]methylene]-6-methoxy-1,3-benzodioxole-5-propanoic acid

### EXAMPLE 13

### (E)-3-[1-n-Butyl-5-[2-[2-carboxyphenyl)methoxy]-4-methoxyphenyl]-1H-pyrazol-4-yl]-2-[(2,3-dimethoxyphenyl)methyl]-prop-2-enoic acid 188-189°C

### EXAMPLE 14

### (E)-3-[1-n-Butyl-5-[2-[2-carboxyphenyl)methoxy]-4-methoxyphenyl]-1H-pyrazol-4-yl]-2-[(2,5-dimethoxyphenyl)methyl]-prop-2-enoic acid 192-194°C

### EXAMPLE 15

### (E)-alpha-[[1-Benzyl-5-[2-[(2-carboxyphenyl)methoxy]-4-methoxyphenyl]-1H-pyrazol-4-yl]methylene]-6-methoxy-1,3-benzodioxole-5-propanoic acid

### EXAMPLE 16

### (E)-3-[1-n-Butyl-5-[2-[2-carboxyphenyl)methoxy]-4-methoxyphenyl]-1H-pyrazol-4-yl]-2-[(2,6-dimethoxyphenyl)methyl]-prop-2-enoic acid

### EXAMPLE 17

### (E)-3-[1-n-Butyl-5-[2-[2-carboxyphenyl)methoxy]-4-methoxyphenyl]-1H-pyrazol-4-yl]-2-[(2,5-dichlorophenyl)methyl]-prop-2-enoic acid 177-179°C

### EXAMPLE 18

### (E)-alpha-[[1-n-Butyl-5-[2-[(2-hydroxymethylphenyl)methoxy]-4-methoxyphenyl]-1H-pyrazol-4-yl]methylene]-6-methoxy-1,3-benzodioxole-5-propanoic acid

### EXAMPLE 19

### (E)-alpha-[[1-n-Butyl-5-[2-[(2-carboxyphenyl)methoxy]-4-methoxyphenyl]-1H-pyrazol-4-yl]methylene]-6-methoxy-1,3-benzodioxan-5-propanoic acid 124-126°C

### EXAMPLE 20

### (E)-alpha-[[5-[2-[(2-Carboxyphenyl)methoxy]-4-methoxyphenyl]-1-cyclopropylmethyl-1H-pyrazol-4-yl]methylene]-6-methoxy-1,3-benzodioxole-5-propanoic acid

### EXAMPLE 21

### (E)-3-[1-n-Butyl-5-[2-(2-carboxyphenyl)methoxy]-4-methoxyphenyl]-1H-pyrazol-4-yl]-2-[(2-methoxy-5-chlorophenyl)]-prop-2-enoic acid 202-203°C

### EXAMPLE 22

### (E)-3-[1-n-Butyl-5-[2-(2-carboxyphenyl)methoxy]-4-methoxyphenyl]-1H-pyrazol-4-yl]-2-[2-chloro-5-methoxyphenyl]-prop-2-enoic acid 141-143°C

### EXAMPLE 23

### (E)-alpha-[[1-n-Butyl-5-[2-[(2-carboxyphenyl)methoxy]-4-methoxyphenyl]-1H-pyrazol-4-yl]methylene-2-chloro-5-thiophene propanoic acid

### EXAMPLE 24

### (E)-alpha-[[1-n-Butyl-5-[2-[(2-carboxyphenyl)methoxy]-4-methoxyphenyl]-1H-pyrazol-4-yl]methylene-3-indolepropanoic acid

### EXAMPLE 25

### (E)-3-[1-n-Butyl-5-[2-[2-(carboxyphenyl)methoxy]-4-methoxyphenyl]-1H-pyrazol-4-yl]-2-[(2,4-dmethoxyphenyl)methyl]-prop-2-enoic acid 197-199°C

### EXAMPLE 26

### (E)-3-[1-n-Butyl-5-[2-(2-carboxyphenyl)methoxy]-4-methoxyphenyl]-1H-pyrazol-4-yl]-2-[(5-fluoro-2-methoxyphenyl)methyl]-prop-2-enoic acid 200-201°C

### EXAMPLE 27

### (E)-3-[1-n-Butyl-5-[2-(2-carboxyphenyl)methoxy]-4-methoxyphenyl]-1H-pyrazol-4-yl]-2-[(2-methoxy-5-nitrophenyl)methyl]-prop-2-enoic acid 239-241°C

### EXAMPLE 28

### (E)-3-[1-n-Butyl-5-[2-(2-carboxyphenyl)methoxy]-4-methoxyphenyl]-1H-pyrazol-4-yl]-2-[(2-methoxyphenyl)methyl]-prop-2-enoic acid 182-183°C

### EXAMPLE 29

### (E)-3-[1-n-Butyl-5-[2-(2-carboxyphenyl)methoxy]-4-methoxyphenyl]-1H-pyrazol-4-yl]-2-[5-benzyloxy-2-methoxyphenyl)methyl]-prop-2-enoic acid 180-183°C

### EXAMPLE 30

### (E)-3-[1-n-Butyl-5-[2-(2-carboxyphenyl)methoxy]-4-methoxyphenyl]-1H-pyrazol-4-yl]-2-[(2-methoxyphenyl-5-methylthio)methyl]-prop-2-enoic acid 158-160°C

### EXAMPLE 31

### (E)-alpha-[[1-n-Butyl-5-[2-[(2-carboxyphenyl)methoxy]-4-methoxyphenyl]-1H-pyrazol-4-yl]methylene]-2,4-dibromo-5-methoxybenzenepropanoic acid

### EXAMPLE 32

### (E)-alpha-[[1-n-Butyl-5-[2-[(2-carboxyphenyl)methoxy]-4-methoxyphenyl]-1H-pyrazol-4-yl]methylene]-4-chloro-2.5-dimethoxybenzenepropanoic acid

### EXAMPLE 33

### (E)-alpha-[[1-(3-Butenyl)-5-[2-[(2-carboxyphenyl)methoxy]-4-methoxyphenyl]-1H-pyrazol-4-yl]methylene]-6-methoxy-1,3-benzodioxole-5-propanoic acid

### EXAMPLE 34

### (E)-alpha-[[5-[2-[(2-Carboxyphenyl)methoxy]-4-methoxyphenyl]-1-cyclopropylethyl-1H-pyrazol-4-yl]methylene]-6-methoxy-1,3-benzodioxole-5-propanoic acid

### EXAMPLE 35

### (E)-alpha-[[1-n-Butyl-5-[2-[N-(phenylsulfonyl)-(N-methyl)methylurea]-4-methoxyphenyl]-1H-pyrazol-4-yl]-methylene]-6-methoxy-1,3-benzodioxan-5-propanoic acid trifluoroacetate salt 58-60°C

### EXAMPLE 36

### (E)-alpha-[[1-n-Butyl-5-[2-(2-carboxyphenylmethylether)-4-methoxyphenyl]-1H-pyrazol-4-yl]methylene]-6-methoxy-1,3-benzodioxan-5-propanoic acid 185-187°C

### EXAMPLE 37

### (E)-3-[1-n-Butyl-5-[2-(2-carboxyphenyl)methoxy-4-methoxyphenyl]-1,2,3-triazol-4-yl]-2-[(2-methoxy-4,5-methylenedioxy)phenylmethyl]-prop-2-enoic acid m.p. 195°C.

### EXAMPLE 38

### (E)-3-[1-n-Butyl-5-[2-(2-hydroxymethyl)phenylmethoxy-4-methoxyphenyl]-1,2,3-triazol-4-yl]-2-[(2-methoxy-4,5-methylenedioxy)phenylmethyl]-prop-2-enoic acid m.p. 134-137°C.

### EXAMPLE 39

Formulations for pharmaceutical use incorporating compounds of the present invention can be prepared in various forms and with numerous excipients. Examples of such formulations are given below.

### Inhalant Formulation

A compound of Formula (I), (1 mg to 100 mg) is aerosolized from a metered dose inhaler to deliver the desired amount of drug per use.

| Tablets/Ingredients | Per Tablet |
|---|---|
| 1. Active ingredient | 40 mg |
| (Cpd of Form. I) | |
| 2. Corn Starch | 20 mg |
| 3. Alginic acid | 20 mg |
| 4. Sodium Alginate | 20 mg |
| 5. Mg stearate | 1.3 mg |
| | 2.3 mg |

### Procedure for tablets:

Step 1 Blend ingredients No. 1, No. 2, No. 3 and No. 4 in a suitable mixer/blender.
Step 2 Add sufficient water portion-wise to the blend from Step 1 with careful mixing after each addition. Such additions of water and mixing until the mass is of a consistency to permit its conversion to wet granules.
Step 3 The wet mass is converted to granules by passing it through an oscillating granulator using a No. 8 mesh (2.38 mm) screen.
Step 4 The wet granules are then dried in an oven at 140°F (60°C) until dry.
Step 5 The dry granules are lubricated with ingredient No. 5.
Step 6 The lubricated granules are compressed on a suitable tablet press.

### Parenteral Formulation

A pharmaceutical composition for parenteral administration is prepared by dissolving an appropriate amount of a compound of formula (I) in polyethylene glycol with heating. This solution is then diluted with water for injections Ph Eur. (to 100 ml). The solution is then steriled by filtration through a 0.22 micron membrane filter and sealed in sterile containers.

## Claims

1. A compound of Formula (I): 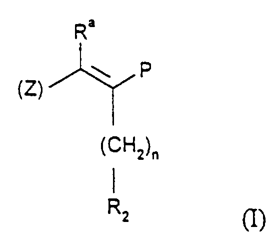 wherein (Z) is 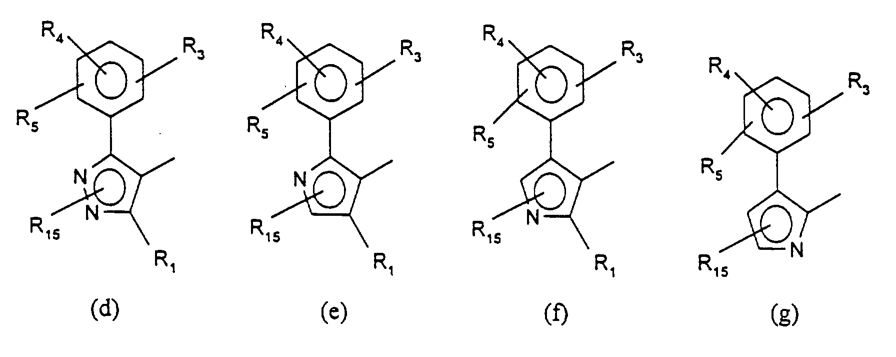 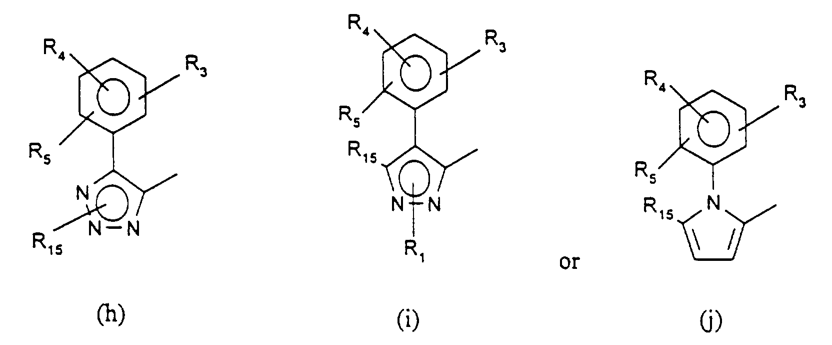
P is tetrazol-5-yl, CO₂R₆ or C(O)N(R₆)S(O)_{q}R₁₀;
R^{a} is independently hydrogen or C₁₋₆alkyl;
R₁ is independently hydrogen, Ar, C₁₋₆alkyl, or C₁₋₆ alkoxy;
R₂ is Ar, C₁₋₈alkyl, C(O)R₁₄ or 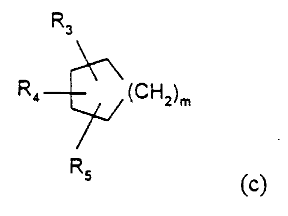
R₃ and R₅ are independently R₁₃OH, C₁₋₈alkoxy, S(O)_{q}R₁₁, N(R₆)₂, NO₂, Br, F, I, Cl, CF₃, NHCOR₆, R₁₃CO₂R₇, -X-R₉-Y, -X(C(R₆)₂)OR₆, -(CH₂)ₘX'R₈ or - X(CH₂)ₙR₈ wherein each methylene group within -X(CH₂)ₙR₈ may be unsubstituted or substituted by one or two -(CH₂)ₙAr groups;
R₄ is independently R₁₁, OH, C₁₋₅alkoxy, S(O)_{q}R₁₁, N(R₆)₂, Br, F, I, Cl or NHCOR₆, wherein the C₁₋₅alkoxy may be unsubstituted or substituted by OH, methoxy or halogen;
R₆ is independently hydrogen or C₁₋₈alkyl;
R₇ is independently hydrogen, C₁₋₁₀alkyl, C₂₋₁₀alkenyl or C₂₋₈alkynyl, all of which may be unsubstituted or substituted by one or more OH, N(R₆)₂, CO₂R₁₂, halogen or XC₁₋₁₀alkyl; or R₇ is (CH₂)ₙAr;
R₈ is independently R₁₁, CO₂R₇, CO₂C(R₁₁)₂O(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, CN, CO₂(CH₂)ₘC(O)N(R₆)₂, C(R₁₁)₂N(R₇)₂, C(O)N(R₆)₂, NR₇C(O)NR₇SO₂R₁₁, OR₆, or tetrazolyl which is unsubstituted or substituted by C₁₋₆alkyl;
R₉ is independently a bond, C₁₋₁₀alkylene, C₁₋₁₀alkenylene, C₁₋₁₀alkylidene, C₁₋ ₁₀alkynylene, all of which may be linear or branched, or phenylene, all of which may be unsubstituted or substituted by one or more OH, N(R₆)₂, COOH or halogen;
R₁₀ is independently C₁₋₁₀alkyl, N(R₆)₂ or Ar;
R₁₁ is independently hydrogen, Ar, C₁₋₈alkyl, C₂₋₈alkenyl, C₂₋₈alkynyl, all of which may be unsubstituted or substituted by one or more OH, CH₂OH, N(R₆)₂ or halogen;
R₁₂ is independently hydrogen, C₁₋₆alkyl, C₂₋₆alkenyl or C₂₋₇alkynyl;
R₁₃ is independently divalent Ar, C₁₋₁₀alkylene, C₁₋₁₀alkylidene, C₂₋₁₀alkenylene, all of which may be unsubstituted or substituted by one or more OH, CH₂OH, N(R₆)₂ or halogen;
R₁₄ is independently hydrogen, C₁₋₁₀alkyl, XC₁₋₁₀alkyl, Ar or XAr;
R₁₅ is independently hydrogen, Ar, C₁₋₆alkyl, or XAr;
R₁₆ is independently C₁₋₆alkyl or phenyl substituted by one or more C₁₋₆alkyl, OH, C₁₋₅alkoxy, S(O)_{q}R₆, N(R₆)₂, Br, F, I, Cl, CF₃ or NHCOR₆;
X is independently (CH₂)ₙ, O, NR₆ or S(O)_{q};
X' is independently O, NR₆ or S(O)_{q};
Y is independently CH₃ or X(CH₂)ₙAr;
Ar is: 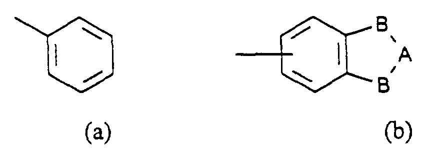 naphthyl, indolyl, pyridyl, thienyl, oxazolidinyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more Z₁ or Z₂ groups;
A is independently C=O, or (C(R₆)₂)ₘ;
B is independently -CH₂- or -O-;
Z₁ and Z₂ are independently hydrogen, XR₆, C₁₋₈alkyl, (CH₂)_{q}CO₂R₆, C(O)N(R₆)₂, CN, (CH₂)ₙOH, NO₂, F, Cl, Br, I, N(R₆)₂, NHC(O)R₆, O(CH₂)ₘC(O)NRₐSO₂R₁₆, (CH₂)ₘOC(O)NRₐSO₂R₁₆, O(CH₂)ₘNRₐC(O)NRₐSO₂R₁₆ or tetrazolyl which may be unsubstituted or substituted by C₁₋₆alkyl, CF₃ or C(O)R₆;
m is independently 1 to 3;
n is independently 0 to 6;
q is independently 0, 1 or 2;
provided R₃, R₄ and R₅ are not O-O(CH₂)ₙAr or O-O R₆;
and further provided that R₂ is not dihydrobenzofuran;
or a pharmaceutically acceptable salt thereof.

2. A compound of Formula (I) wherein P is CO₂R₆; R₁ is hydrogen; R₂ is Ar, cyclohexyl or C₁₋₄alkyl; R₃ and R₅ are independently hydrogen, CO₂R₆, OH, C₁₋₈alkoxy, C₁₋₈alkyl, N(R₆)₂, NO₂, Br, F, Cl, I, R₁₃CO₂R₇, X(CH₂)ₙR₈, (CH₂)ₘX'R₈, or X(C(R₆)₂)ₘOR₆; R₄ is hydrogen, OH, C₁₋₅alkoxy, N(R₆)₂, Br, F, Cl, I, NHCOCH₃, or S(O)_{q} C₁₋₅alkyl wherein the C₁₋₅alkyl may be unsubstituted or substituted by OH, methoxy or halogen; R₆ is hydrogen, methyl or ethyl; R₇ is hydrogen, C₁₋₁₀alkyl, C₂₋₁₀ alkenyl or C₂₋₈alkynyl, all of which may be unsubstituted or substituted by one or more OH, N(R₆)₂, CO₂R₁₂, halogen, or R₇ is (CH₂)ₙAr wherein n is zero or 1 and Ar is substituted phenyl; R₁₁ is hydrogen, phenyl, or pyridyl all of which may be unsubstituted or substituted by one or two C₁₋₄alkyl groups; C₁₋₈alkyl, C₂₋₈alkenyl, or C₂₋₈alkynyl, all of which may be unsubstituted or substituted by one or more OH, CH₂OH, N(R₆)₂, or halogen; R₁₂ is hydrogen or C₁₋₆alkyl; R₁₃ is phenylene, pyridylene, or C₂₋₁₀alkylene, all of which may be unsubstituted or substituted by one or more CO₂R₆, OH, CH₂OH, N(R₆)₂, or halogen; R₁₅ is hydrogen or C₁₋₆alkyl; and (Z) is (d).

3. A compound of claim 2 wherein P is CO₂H; R₁ is hydrogen; R₂ is a group Ar wherein Ar is a group (a) or (b) and in said group (a) or (b), Z₁ and Z₂ are independently hydrogen, CO₂R₆, (CH₂)ₙOH, C₁₋₄alkyl or C₁₋₆ alkoxy and A is CH₂, and both Bs are O; R₃ is Br, Cl, C₁₋₈alkoxy or X(CH₂)ₙR₈, wherein X is O, n is 0, 1, or 2, and R₈ is selected from: CO₂H, OH, tetrazolyl optionally substituted by C₁₋₈alkyl; CONR₇SO₂R₁₁ wherein R₇ is H or C₁₋₈alkyl, R₁₁ is C₁₋₈alkyl or phenyl optionally substituted by Br, Cl, F, C₁₋₈alkyl; or R₈ is phenyl or pyridyl substituted by one or more Br, Cl, CO₂H, CH₂OH; R₅ is methoxy or N(R₆)₂ wherein R₆ is H or methyl; R₄ is hydrogen; R₆ is hydrogen, methyl or ethyl; R₇ is hydrogen, C₁₋₁₀alkyl, C₂₋₁₀alkenyl or C₂₋₈alkynyl, all of which may be unsubstituted or substituted by one or more OH, N(R₆)₂, CO₂R₁₂, or halogen, or R₇ is (CH₂)ₙAr wherein R₇ is (CH₂)ₙAr and n is zero or 1 and Ar is phenyl unsubstituted or substituted by halogen or C₁₋₅ alkoxy; R₁₁ is hydrogen, phenyl, or pyridyl wherein the phenyl or pyridyl may be unsubstituted or substituted by one or two C₁₋₄alkyl groups; C₁₋₈alkyl, C₂₋₈alkenyl, or C₂₋₈alkynyl, all of which may be unsubstituted or substituted by one or more OH, CH₂OH, N(R₆)₂, or halogen; R₁₂ is hydrogen or C₁₋₆alkyl; R₁₃ is phenylene, pyridylene, or C₂₋₁₀alkylene, all of which may be unsubstituted or substituted by one or more CO₂R₆, OH, CH₂OH, N(R₆)₂, or halogen; R₁₅ is hydrogen, ethyl, isopropyl, *n*-butyl, cyclopropylmethyl or cyclopropylethyl; and (Z) is (d).

4. A compound of claim 1 selected from:
(E)-alpha-[[5-[2-[(2-carboxyphenyl)methoxy]-4-methoxyphenyl]-1-ethyl-1H-pyrazol-4-yl]methylene]-6-methoxy-1,3-benzodioxole-5-propanoic acid;
(E)-alpha-[[1-Allyl-5-[2-[(2-carboxyphenyl)methoxy]-4-methoxyphenyl]-1H-pyrazol-4-yl]methylene]-6-methoxy-1,3-benzodioxole-5-propanoic acid;
(E)-alpha-[[1-Butyl-5-[2-[(2-carboxyphenyl)methoxy]-4-chlorophenyl]-1H-pyrazol-4-yl]methylene]-6-methoxy-1,3-benzodioxole-5-propanoic acid;
(E)-alpha-[[1-Butyl-5-[4-methoxy-2-[[N-(phenylsulfonyl)]carboxamidomethoxy]phenyl]-1H-pyrazol-4-yl]methylene]-6-methoxy-1,3-benzodioxole-5-propanoic acid;
(E)-3-[1-Butyl-5-[2-[(2-carboxyphenyl)methoxy]-4-methoxyphenyl]-1H-pyrazol-4-yl]-2-[(2,5-dimethoxyphenyl)methyl]-prop-2-enoic acid;
(E)-alpha-[[1-Butyl-5-[2-[(2-hydroxymethylphenyl)methoxy]-4-methoxyphenyl]-1H-pyrazol-4-yl]methyiene]-6-methoxy-1,3-benzodioxole-5-propanoic acid;
(E)-alpha-[[1-Butyl-5-[2-[(2-carboxyphenyl)methoxy]-4-methoxyphenyl]-1H-pyrazol-4-yl]methylene]-7-methoxy-1,4-benzodioxan-6-propanoic acid;
(E)-alpha-[[5-[2-[(2-Carboxyphenyl)methoxy]-4-methoxyphenyl]-1-cyclopropylmethyl-1H-pyrazol-4-yl]methylene]-6-methoxy-1,3-benzodioxole-5-propanoic acid;
(E)-alpha-[[1-(3-Butenyl)-5-[2-[(2-hydroxymethylphenyl)methoxy]-4-methoxyphenyl]-1H-pyrazol-4-yl]methylene]-6-methoxy-1,3-benzodioxole-5-propanoic acid;
(E)-alpha-[[5-[2-[(2-Carboxyphenyl)methoxy]-4-methoxyphenyl]-1-cyclopropylethyl-1H-pyrazol-4-yl]methylene]-6-methoxy-1,3-benzodioxole-5-propanoic acid;
(E)-alpha-[[1-Butyl-5-[2-[(2-carboxyphenoxy)methyl]-4-methoxyphenyl]-1H-pyrazol-4-yl]methylene]-6-methoxy-1,3-benzodioxole-5-propanoic acid.

5. A compound of claim 1 which is (E)-alpha-[[1-Butyl-5-[2-[(2-carboxyphenyl)methoxy]-4-methoxyphenyl]-1H-pyrazol-4-yl]methylene]-6-methoxy-1,3-benzodioxole-5-propanoic acid.

6. A compound of claim 5 which is the disodium salt thereof.

7. A pharmaceutical composition comprising a compound of any of claims 1 to 6 and a pharmaceutically acceptable carrier.

8. A compound of any of claims 1 to 6 for use as an endothelin receptor antagonist.

9. Use of a compound of Formula (I) according to any of claims 1 to 6 in the manufacture of a medicament for the treatment of a disease caused by an excess of endothelin.

10. Use of a compound according to claim 9 wherein the disease is renal failure or cerebrovascular disease.

11. Use of a compound according to claim 9 in the manufacture of a medicament for the prophylaxis or treatment of radiocontrast induced renal failure.

12. Use of a compound according to claim 9 wherein the disease is congestive heart failure.

13. Use of a compound according to claim 9 wherein the disease is unstable angina, coronary vasospasm and myocardial salvage.

14. Use of a compound according to claim 9 in the manufacture of a medicament for preventing or treating restenosis.

15. Use of a compound according to claim 9 wherein the disease is pulmonary hypertension.

16. Use of a compound according to claim 9 wherein the disease is stroke or subarachnoid hemorrhage.

17. A process for preparing a compound of Formula (Id) by:
(a) Reaction of a compound of Formula (II): 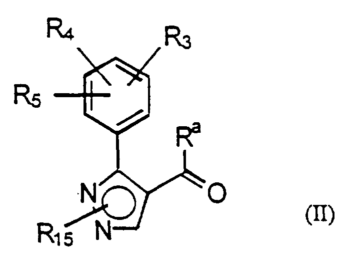 or a protected form or precursor thereof with a compound of Formula (8); 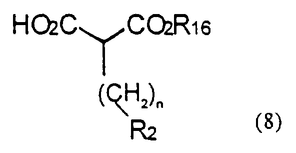 wherein R₂ and R₁₆ are as defined for Formula (Id) in claim 1;
followed if necessary or desired by:
(b) conversion of one compound of Formula (Id) into a different compound of Formula (Id) e.g.
(i) when Formula (Id) contains a group CO₂R₆, CO₂R₇ or CO₂R₁₂ wherein R₆, R₇ or R₁₂ is alkyl, conversion to a corresponding compound where R₆, R₇ or R₁₂ represents hydrogen;
(ii) when Formula (Id) contains a hydroxy group (e.g. in R₃, R₄ or R₅) conversion to a different group, e.g. a group (CH₂)Ar where Ar is optionally substituted phenyl, by a method well known in the art; and/or
(c) salt formation.

## Patentansprüche

1. Verbindung der Formel (I): 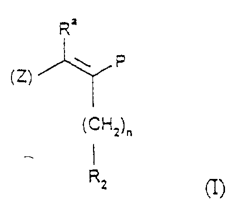 wobei (Z) 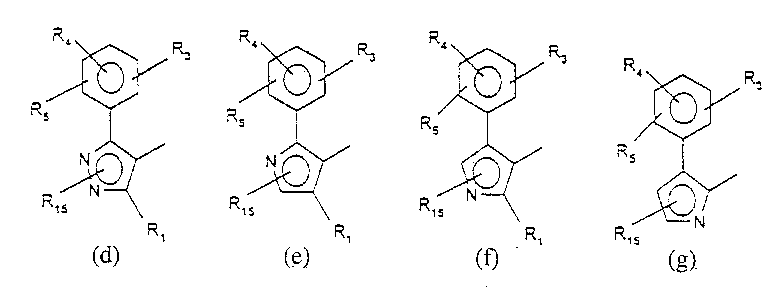 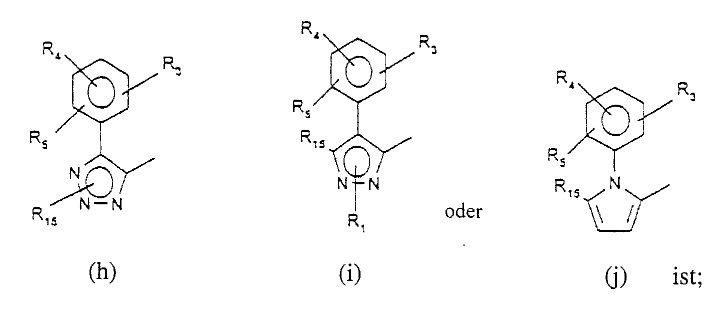
P die Bedeutung Tetrazol-5-yl, CO₂R₆ oder C(O)N(R₆)S(O)_{q}R₁₀ hat;
R^{a} unabhängig ein Wasserstoffatom oder ein C₁₋₆-Alkylrest ist;
R₁ unabhängig ein Wasserstoffatom, Ar, ein C₁₋₆-Alkyl- oder C₁₋₆-Alkoxyrest ist;
R₂ Ar, ein C₁₋₈-Alkylrest, ein Rest C(O)R₁₄ oder 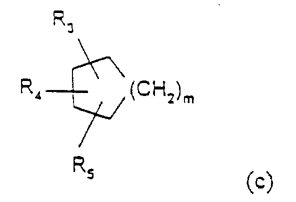 ist;
R₃ und R₅ unabhängig die Bedeutung R₁₃OH, C₁₋₈-Alkoxy, S(O)_{q}R₁₁, N(R₆)₂, NO₂, Br, F, I, Cl, CF₃, NHCOR₆, R₁₃CO₂R₇, -X-R9-Y, -X(C(R₆)₂OR₆, -(CH₂)ₘX'R₈ oder -X(CH₂)ₙR₈ haben, wobei jede Methylengruppe innerhalb des Rests -X(CH₂)ₙR₈ unsubstituiert oder durch ein oder zwei Reste -(CH₂)ₙAr substituiert sein kann;
R₄ unabhängig die Bedeutung R₁₁, OH, C₁₋₅-Alkoxy, S(O)_{q}R₁₁, N(R₆)₂, Br, F, I, Cl oder NHCOR₆ hat, wobei der C₁₋₅-Alkoxyrest unsubstituiert oder durch OH, eine Methoxygruppe oder ein Halogenatom substituiert sein kann;
R₆ unabhängig ein Wasserstoffatom oder ein C₁₋₈-Alkylrest ist;
R₇ unabhängig ein Wasserstoffatom, C₁₋₁₀-Alkyl-, C₂₋₁₀-Alkenyl- oder C₂₋₈-Alkinylrest ist, die jeweils unsubstituiert oder durch eine oder mehrere Gruppen OH, N(R₆)₂, CO₂R₁₂, Halogenatome oder XC₁₋₁₀-Alkylreste substituiert sein können; oder R₇ ein Rest (CH₂)ₙAr ist;
R₈ unabhängig die Bedeutung R₁₁, CO₂R₇, CO₂C(R₁₁)₂O(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, CN, CO₂(CH₂)ₘC(O)N(R₆)₂, C(R₁₁)₂N(R₇)₂, C(O)N(R₆)₂, NR₇C(O)NR₇SO₂R₁₁, OR₆ hat, oder eine Tetrazolylgruppe, die unsubstituiert oder durch einen C₁₋₆-Alkylrest substituiert ist, ist;
R₉ unabhängig eine Bindung, ein C₁₋₁₀-Alkylen-, C₁₋₁₀-Alkenylen-, C₁₋₁₀-Alkyliden-, C₁₋₁₀-Alkinylenrest, die jeweils linear oder verzweigt sein können, oder eine Phenylengruppe ist, die jeweils unsubstituiert oder durch eine oder mehrere Gruppen OH, N(R₆)₂, COOH oder Halogenatome substituiert sein können;
R₁₀ unabhängig ein C₁₋₁₀-Alkylrest, N(R₆)₂ oder Ar ist;
R₁₁ unabhängig ein Wasserstoffatom, Ar, ein C₁₋₈-Alkyl-, C₂₋₈-Alkenyl-, C₂₋₈-Alkinylrest ist, die jeweils unsubstituiert oder durch eine oder mehrere Gruppen OH, CH₂OH, N(R₆)₂ oder Halogenatome substituiert sein können;
R₁₂ unabhängig ein Wasserstoffatom, C₁₋₆-Alkyl-. C₂₋₆-Alkenyl- oder C₂₋₇-Alkinylrest ist;
R₁₃ unabhängig zweiwertiges Ar, ein C₁₋₁₀-Alkylen-, C₁₋₁₀-Alkyliden-, C₂₋₁₀-Alkenylen ist, die jeweils unsubstituiert oder durch eine oder mehrere Gruppen OH, CH₂OH, N(R₆)₂ oder Halogenatome sein können;
R₁₄ unabhängig ein Wasserstoffatom, ein C₁₋₁₀-Alkyl-, XC₁₋₁₀-Alkylrest, Ar oder XAr ist;
R₁₅ unabhängig ein Wasserstoffatom, Ar, ein C₁₋₆-Alkylrest oder XAr ist;
R₁₆ unabhängig ein C₁₋₆-Alkylrest oder eine Phenylgruppe ist, die durch eine oder mehrere Gruppen C₁₋₆-Alkyl, OH, C₁₋₅-Alkoxy, S(O)_{q}R₆, N(R₆)₂, Br, F, I, Cl, CF₃ oder NHCOR₆ substituiert ist;
X unabhängig die Bedeutung (CH₂)ₙ, O, NR₆ oder S(O)_{q} hat;
X' unabhängig die Bedeutung O, NR₆ oder S(O)_{q} hat;
Y unabhängig CH₃ oder X(CH₂)ₙAr ist;
Ar: 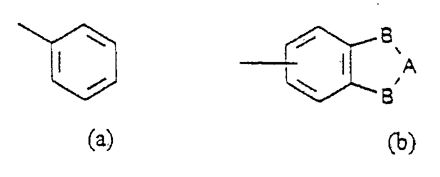 Naphthyl, Indolyl, Pyridyl, Thienyl, Oxazolidinyl, Thiazolyl, Isothiazolyl, Pyrazolyl, Triazolyl, Tetrazolyl, Imidazolyl, Imidazolidinyl, Thiazolidinyl, Isoxazolyl, Oxadiazolyl, Thiadiazolyl, Morpholinyl, Piperidinyl, Piperazinyl, Pyrrolyl oder Pyrimidyl ist; die jeweils unsubstituiert oder durch eine oder mehrere Gruppen Z₁ oder Z₂ substituiert sind.
A unabhängig die Bedeutung C=O oder (C(R₆)₂)ₘ hat;
B unabhängig die Bedeutung -CH2- oder -O- hat;
Z₁ und Z₂ unabhängig ein Wasserstoffatom, XR₆, ein C₁₋₈-Alkylrest, (CH₂)_{q}CO₂R₆, C(O)N(R₆)₂, CN, (CH₂)ₙOH, NO₂, F, Cl, Br, I, N(R₆)₂, NHC(O)R₆, O(CH₂)ₘC(O)NRₐSO₂R₁₆, (CH₂)ₘOC(O)NRₐSO₂R₁₆, O(CH₂)ₘNRₐC(O)NRₐSO₂R₁₆ oder Tetrazolyl, das unsubstituiert oder durch C₁₋₆-Alkyl, CF₃ oder C(O)R₆ substituiert ist, ist;
m unabhängig 1 bis 3 ist;
n unabhängig 0 bis 6 ist;
q unabhängig 0, 1 oder 2 ist;
mit der Maßgabe, dass R₃, R₄ und R₅ nicht O-O(CH₂)ₙAr oder O-O-R₆ sind; und mit der weiteren Maßgabe, dass R₂ keine Dihydrobenzofurangruppe ist; oder ein pharmazeutisch verträgliches Salz davon.

2. Verbindung der Formel (I), wobei P die Bedeutung CO₂R₆ hat; R₁ ein Wasserstoffatom ist; R₂ Ar, eine Cyclohexylgruppe oder ein C₁₋₄-Alkylrest ist; R₃ und R₅ unabhängig ein Wasserstoffatom, CO₂R₆, OH, C₁₋₈-Alkoxy, C₁₋₈-Alkyl, N(R₆)₂, NO₂, Br, F, Cl, I, R₁₃CO₂R₇, X(CH₂)ₙR₈, (CH₂)ₘX'R₈ oder X(C(R₆)₂)ₘOR₆ ist; R₄ ein Wasserstoffatom, OH, C₁₋₅-Alkoxy, N(R₆)₂, Br, F, Cl, I, NHCOCH₃ oder S(O)_{q}C₁₋₅-Alkyl ist, wobei der C₁₋₅-Alkylrest unsubstituiert oder durch eine Gruppe OH, eine Methoxygruppe oder ein Halogenatom substituiert sein kann; R₆ ein Wasserstoffatom, eine Methyl- oder Ethylgruppe ist; R₇ ein Wasserstoffatom, ein C₁₋₁₀-Alkyl-, C₂₋₁₀-Alkenyl- oder C₂₋₈-Alkinylrest ist, die jeweils unsubstituiert oder durch eine oder mehrere Gruppen OH, N(R₆)₂, CO₂R₁₂, Halogenatome substituiert sein können, oder R₇ die Bedeutung (CH₂)ₙAr hat, wobei n gleich Null oder 1 ist und Ar eine substituierte Phenylgruppe ist; R₁₁ ein Wasserstoffatom, eine Phenyl- oder Pyridylgruppe, die jeweils unsubstituiert oder durch eine oder zwei C₁₋₄-Alkylreste substituiert sein können; ein C₁₋₈-Alkyl-, C₂₋₈-Alkenyl- oder C₂₋₈-Alkinylrest, die jeweils unsubstituiert oder durch ein oder mehrere Gruppen OH, CH₂OH, N(R₆)₂ oder Halogenatome substituiert sein können, ist; R₁₂ ein Wasserstoffatom oder ein C₁₋₆-Alkylrest ist; R₁₃ eine Phenylen-, Pyridylen- oder C₂₋₁₀-Alkylen-Gruppe ist, die jeweils unsubstituiert oder durch eine oder mehrere Gruppen CO₂R₆, OH, CH₂OH, N(R₆)₂ oder Halogenatome substituiert sein können; R₁₅ ein Wasserstoffatom oder C₁₋₆-Alkylrest ist; und (Z) die Bedeutung (d) hat.

3. Verbindung nach Anspruch 2, wobei P die Bedeutung CO₂H hat, R₁ ein Wasserstoffatom ist; R₂ ein Rest Ar ist, wobei Ar eine Gruppe (a) oder (b) ist und in dieser Gruppe (a) oder (b) Z₁ und Z₂ unabhängig voneinander ein Wasserstoffatom, CO₂R₆, (CH₂)ₙOH, C₁₋₄-Alkyl oder C₁₋₆-Alkoxy ist und A die Bedeutung CH₂ hat, und beide Reste B die Bedeutung O haben; R₃ die Bedeutung Br, Cl, C₁₋₈-Alkoxy oder X(CH₂)ₙR₈ hat, wobei X die Bedeutung O hat, n gleich 0, 1 oder 2 ist und R₈ ausgewählt ist aus: CO₂H, OH, Tetrazolyl, das gegebenenfalls durch einen C₁₋₈-Alkylrest substituiert ist; CONR₇SO₂R₁₁, wobei R₇ die Bedeutung H oder C₁₋₈-Alkyl hat, R₁₁ ein C₁₋₈-Alkylrest oder eine Phenylgruppe ist, die gegebenenfalls durch Br, Cl, F, C₁₋₈-Alkyl substituiert ist; oder R₈ eine Phenyl- oder Pyridylgruppe ist, die durch eine oder mehrere Gruppen Br, Cl, CO₂H, CH₂OH substituiert sind; R₅ Methoxy oder N(R₆)₂ ist, wobei R₆ die Bedeutung H oder Methyl hat; R₄ ein Wasserstoffatom ist; R₆ ein Wasserstoffatom, eine Methyl- oder Ethylgruppe ist; R₇ ein Wasserstoffatom, ein C₁₋₁₀-Alkyl-, C₂₋₁₀-Alkenyl- oder C₂₋₈-Akinylrest ist, die jeweils unsubstituiert oder durch eine oder mehrere Gruppen OH, N(R₆)₂, CO₂R₁₂ oder Halogenatome substituiert sind, oder R₇ eine Gruppe (CH₂)ₙAr ist, wobei R₇ die Bedeutung (CH₂)ₙAr hat und n gleich Null oder 1 ist und Ar eine Phenylgruppe ist, die unsubstituiert oder durch ein Halogenatom oder einen C₁₋₅-Alkoxyrest substituiert ist; R₁₁ ein Wasserstoffatom, eine Phenyl- oder Pyridylgruppe, wobei die Phenyl- oder Pyridylgruppe unsubstituiert oder durch eine oder zwei C₁₋₄-Alkylreste substituiert sein kann; ein C₁₋₈-Alkyl, C₂₋₈-Alkenyl oder C₂₋₈-Alkinyl, die jeweils unsubstituiert oder durch eine oder mehrere Gruppen OH, CH₂OH, N(R₆)₂ oder Halogenatome sein können, ist; R₁₂ ein Wasserstoffatom oder ein C₁₋₆-Alkylrest ist; R₁₃ eine Phenylen-, Pyridylen- oder C₂₋₁₀-Alkylen-Gruppe ist, die jeweils unsubstituiert oder durch eine oder mehrere Gruppen CO₂R₆, OH, CH₂OH, N(R₆)₂ oder Halogenatome substituiert sein können; R₁₅ ein Wasserstoffatom, eine Ethyl-, Isopropyl-, n-Butyl-, Cyclopropylmethyl-, oder Cyclopropylethylgruppe ist; und Z die Bedeutung (d) hat.

4. Verbindung nach Anspruch 1, ausgewählt aus:
(E)-alpha-[[5-[2-[(2-Carboxyphenyl)methoxy]-4-methoxyphenyl]-1-ethyl-1H-pyrazol-4-yl]methylen]-6-methoxy-1,3-benzodioxol-5-propansäure;
(E)-alpha-[[1-Allyl-5-[2-[(2-carboxyphenyl)methoxy]-4-methoxyphenyl]-1H-pyrazol-4-yl]methylen]-6-methoxy-1,3-benzodioxol-5-propansäure;
(E)-alpha-[[1-Butyl-5-[2-[(2-carboxyphenyl)methoxy]-4-chlorphenyl]-1H-pyrazol-4-yl]methylen]-6-methoxy-1,3-benzodioxol-5-propansäure;
(E)-alpha-[[1-Butyl-5-[4-methoxy-2-[[N-(phenylsulfonyl)]carboxamidomethoxy]phenyl]-1H-pyrazol-4-yl]methylen]-6-methoxy-1,3-benzodioxol-5-propansäure;
(E)-3-[1-Butyl-5-[2-[(2-carboxyphenyl)methoxy]-4-methoxyphenyl]-1H-pyrazol-4-yl]-2-[(2,5-dimethoxyphenyl)methyl]-prop-2-ensäure
(E)-alpha-[[1-Butyl-5-[2-[(2-hydroxymethylphenyl)methoxy]-4-methoxyphenyl]-1H-pyrazol-4-yl]methylen]-6-methoxy-1,3-benzodioxol-5-propansäure;
(E)-alpha-[[1-Butyl-5-[2-[(2-carboxyphenyl)methoxy]-4-methoxyphenyl]-1H-pyrazol-4-yl]methylen]-7-methoxy-1,4-benzodioxan-6-propansäure;
(E)-alpha-[[5-[2-[(2-Carboxyphenyl)methoxy]-4-methoxyphenyl]-1-cyclopropylmethyl-1H-pyrazol-4-yl]methylen]-6-methoxy-1,3-benzodioxol-5-propansäure;
(E)-alpha-[[1-(3-Butenyl)-5-[2-[(2-hydroxymethylphenyl)methoxy]-4-methoxyphenyl]-1H-pyrazol-4-yl]methylen]-6-methoxy-1,3-benzodioxol-5-propansäure;
(E)-alpha-[[5-[2-[(2-Carboxyphenyl)methoxy]-4-methoxyphenyl]-1-cyclopropylethyl-1H-pyrazol-4-yl]methylen]-6-methoxy-1,3-benzodioxol-5-propansäure;
(E)-alpha-[[1-Butyl-5-[2-[(2-carboxyphenoxy)methyl]-4-methoxyphenyl]-1H-pyrazol-4-yl]methylen]-6-methoxy-1,3-benzodioxol-5-propansäure.

5. Verbindung nach Anspruch 1, nämlich (E)-alpha-[[1-Butyl-5-[2-[(2-carboxyphenyl)methoxy]-4-methoxyphenyl]-1H-pyrazol-4-yl]methylen]-6-methoxy-1,3-benzodioxol-5-propansäure;

6. Verbindung nach Anspruch 5, welche das Dinatriumsalz ist.

7. Arzneimittel, umfassend eine Verbindung nach einem der Ansprüche 1 bis 6 und einen pharmazeutisch verträglichen Träger.

8. Verbindung nach einem der Ansprüche 1 bis 6 zur Verwendung als Endothelin-Rezeptorantagonist.

9. Verwendung einer Verbindung der Formel (I) nach einem der Ansprüche 1 bis 6 zur Herstellung eines Medikamentes zur Behandlung einer Erkrankung, die durch einen Endothelin-Überschuss verursacht wird.

10. Verwendung einer Verbindung nach Anspruch 9, wobei die Erkrankung Nierenversagen oder Cerebrovaskular-Erkrankung ist.

11. Verwendung einer Verbindung nach Anspruch 9 zur Herstellung eines Medikamentes zur Prophylaxe oder Behandlung von Nierenversagen, das durch ein Röntgenkontrastmittel induziert wird.

12. Verwendung einer Verbindung nach Anspruch 9, wobei die Erkrankung Stauungsherzversagen ist.

13. Verwendung einer Verbindung nach Anspruch 9, wobei die Erkrankung instabile Angina, Koronar-Vasospasmus und Myokard-Wiederherstellung ist.

14. Verwendung einer Verbindung nach Anspruch 9 bei der Herstellung eines Medikamentes zur Vorbeugung oder Behandlung von Restenose.

15. Verwendung einer Verbindung nach Anspruch 9, wobei die Erkrankung pulmonäre Hypertonie ist.

16. Verwendung einer Verbindung nach Anspruch 9, wobei die Erkrankung Herzinfarkt oder subarachnoide Hämorrhagie ist.

17. Verfahren zur Herstellung einer Verbindung der Formel (Id) durch:
(a) Umsetzen einer Verbindung der Formel (II): 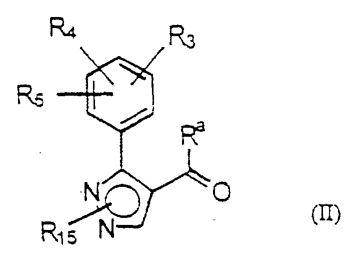 oder einer geschützten Form oder Vorstufe mit einer Verbindung der Formel (8): 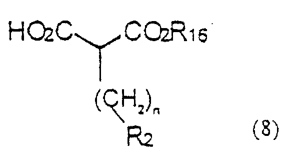 wobei R₂ und R₁₆ die für Formel (Id) in Anspruch 1 definierte Bedeutung haben, gefolgt - wenn nötig oder gewünscht - von:
(b) Umwandeln einer Verbindung der Formel (Id) in eine andere Verbindung der Formel (Id), bspw.
(i) wenn die Formel (Id) eine Gruppe CO₂R₆, CO₂R₇ oder CO₂R₁₂ enthält, wobei R₆, R₇ oder R₁₂ ein Alkylrest ist, Umwandeln in eine entsprechende Verbindung, wobei R₆, R₇ oder R₁₂ ein Wasserstoffatom darstellt;
(ii) wenn Formel (Id) eine Hydroxygruppe (bspw. in R₃, R₄ oder R₅) enthält, Umwandlung in eine andere Gruppe, bspw. eine Gruppe (CH₂)Ar, wobei Ar eine gegebenenfalls substituierte Phenylgruppe ist, durch ein im Fachgebiet bekanntes Verfahren; und/oder
(c) Salzbildung.

## Revendications

1. Composé de formule (I) : 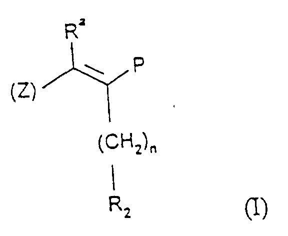 dans laquelle (Z) représente un groupe : 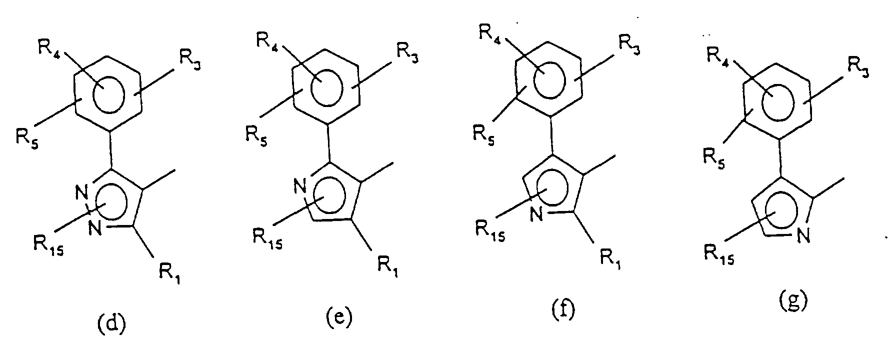 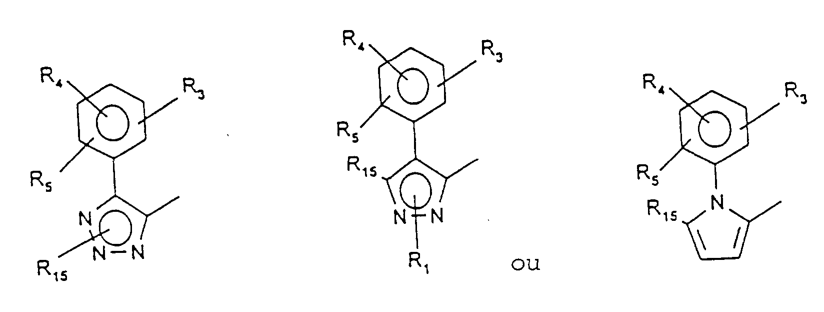
P représente un groupe tétrazole-5-yle, CO₂R₆ ou C(O)N(R₆)S(O)_{q}R₁₀ ;
R^{a} représente indépendamment l'hydrogène ou un groupe alkyle en C₁ à C₆ ;
R₁ représente indépendamment l'hydrogène, un groupe Ar, alkyle en C₁ à C₆ ou alkoxy en C₁ à C₆ ;
R₂ représente un groupe Ar, alkyle en C₁ à C₈, C(O)R₁₄, ou 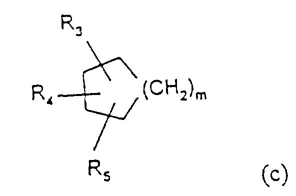
R₃ et R₅ représentent indépendamment un groupe R₁₃OH, alkoxy en C₁ à C₈, S(O)_{q}R₁₁, N(R₆)₂, NO₂, Br, F, I, Cl, CF₃, NHCOR₆, R₁₃CO₂R₇, -X-R₉-Y, -X(C(R₆)₂)OR₆- (CH₂)ₘX'R₈ ou -X(CH₂)ₙR₈, dans lequel chaque groupe méthylène dans le groupe -X(CH₂)ₙR₈ peut être non substitué ou substitué avec un ou deux groupes -(CH₂)ₙAr ;
R₄ représente indépendamment un groupe R₁₁, OH, alkoxy en C₁ à C₅, S(O)_{q}R₁₁, N(R₆)₂, Br, F, I, Cl ou NHCOR₆, le groupe alkoxy en C₁ à C₅ pouvant être non substitué ou substitué avec un substituant OH, méthoxy ou halogéno ;
R₆ représente indépendamment l'hydrogène ou un groupe alkyle en C₁ à C₈ ;
R₇ représente indépendamment l'hydrogène, un groupe alkyle en C₁ à C₁₀, alcényle en C₂ à C₁₀ ou alcynyle en C₂ à C₈, tous ces groupes pouvant être non substitués ou substitués avec un ou plusieurs groupes OH, N(R₆)₂, CO₂R₁₂, halogéno ou X(alkyle en C₁ à C₁₀); ou bien R₇ représente un groupe (CH₂)ₙAr ;
R₈ représente indépendamment un groupe R₁₁, CO₂R₇, CO₂C(R₁₁)₂O(CO)XR₇, PO₃(R₇)₂, SO₂NR₇R₁₁, NR₇SO₂R₁₁, CONR₇SO₂R₁₁, SO₃R₇, SO₂R₇, R(O) (OR₇)R₇, CN, CO₂(CH₂)ₘC(O)N(R₆)₂, C (R₁₁)₂N(R₇)₂, C(O)N(R₆)₂, NR₇C(O)NR₇SO₂R₁₁, OR₆ ou tétrazolyle qui est non substitué ou substitué avec un groupe alkyle en C₁ à C₆ ;
R₉ représente indépendamment une liaison, un groupe alkylène en C₁ à C₁₀, alcénylène en C₁ à C₁₀, alkylidène en C₁ à C₁₀, alcynylène en C₁ à C₁₀, tous ces groupes pouvant être linéaires ou ramifiés, ou un groupe phénylène, tous ces groupes pouvant être non substitués ou substitués avec un ou plusieurs groupes OH, N(R₆)₂, COOH ou halogéno ;
R₁₀ représente indépendamment un groupe alkyle en C₁ à C₁₀, N(R₆)₂ ou Ar ;
R₁₁ représente indépendamment l'hydrogène, un groupe Ar, un groupe alkyle en C₁ à C₈, alcényle en C₂ à C₈, alcynyle en C₂ à C₈, tous ces groupes pouvant être non substitués ou substitués avec un ou plusieurs substituants OH, CH₂OH, N(R₆)₂ ou halogéno ;
R₁₂ représente indépendamment l'hydrogène, un groupe alkyle en C₁ à C₆, alcényle en C₂ à C₆ ou alcynyle en C₂ à C₇ ;
R₁₃ représente indépendamment un groupe Ar divalent, alkylène en C₁ à C₁₀, alkylidène en C₁ à C₁₀, alcénylène en C₂ à C₁₀, tous ces groupes pouvant être non substitués ou substitués avec un ou plusieurs groupes OH, CH₂OH, N(R₆)₂ ou halogéno ;
R₁₄ représente indépendamment l'hydrogène, un groupe alkyle en C₁ à C₁₀, X(alkyle en C₁ à C₁₀), Ar, ou XAr ;
R₁₅ représente indépendamment l'hydrogène, un groupe Ar, alkyle en C₁ à C₆ ou XAr ;
R₁₆ représente indépendamment un groupe alkyle en C₁ à C₆ ou phényle substitué avec un ou plusieurs groupes alkyle en C₁ à C₆, OH, alkoxy en C₁ à C₅, S(O)_{q}R₆, N(R₆)₂, Br, F, I, Cl, CF₃ ou NHCOR₆ ;
X représente indépendamment un groupe (CH₂)ₙ, O, NR₆ ou S(O)_{q} ;
X' représente indépendamment un groupe O, NR₆ ou S(O)_{q} ;
Y représente indépendamment un groupe CH₃ ou X(CH₂)ₙAr ;
Ar représente un groupe : 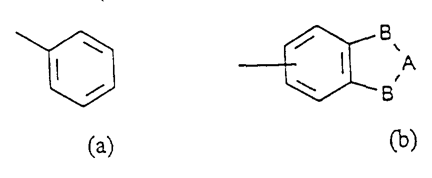 naphtyle, indolyle, pyridyle, thiényle, oxazolidinyle, thiazolyle, isothiazolyle, pyrazolyle, triazolyle, tétrazolyle, imidazolyle, imidazolidinyle, thiazolidinyle, isoxazolyle, oxadiazolyle, thiadiazolyle, morpholinyle, pipéridinyle, pipérazinyle, pyrrolyle, ou pyrimidyle ; tous ces groupes pouvant être non substitués ou substitués avec un ou plusieurs groupes Z₁ ou Z₂ ;
A représente indépendamment un groupe C=O ou (C(R₆)₂)ₘ ;
B représente indépendamment un groupe -CH₂- ou -O- ;
Z₁ et Z₂ représentent indépendamment l'hydrogène, un groupe XR₆, alkyle en C₁ à C₈, (CH₂)_{q}CO₂R₆, C(O)N(R₆)₂, CN, (CH₂)ₙOH, NO₂, F, Cl, Br, I, N(R₆)₂, NHC(O)R₆, O(CH₂)ₘC(O)NRₐSO₂R₁₆, (CH₂)ₘOC (O) NRₐSO₂R₁₆, O(CH₂)ₘNRₐC(O)NRₐSO₂R₁₆ ou tétrazolyle qui peut être non substitué ou substitué avec un groupe alkyle en C₁ à C₆, CF₃ ou C(O)R₆ ;
m a indépendamment une valeur de 1 à 3 ;
n a indépendamment une valeur de 0 à 6 ;
q a indépendamment la valeur 0, 1 ou 2 ;
sous réserve que R₃, R₄ et R₅ ne représentent pas des groupes O-O(CH₂)ₙAr ou O-OR₆ ;
et sous réserve en outre que R₂ ne représente pas un groupe dihydrobenzofuranne ;
ou un de ses sels pharmaceutiquement acceptable.

2. Composé de formule (I) dans laquelle P représente un groupe CO₂R₆ ; R₁ représente l'hydrogène ; R₂ représente un groupe Ar, cyclohexyle ou alkyle en C₁ à C₄ ; R₃ et R₅ représentent indépendamment l'hydrogène, un groupe CO₂R₆, OH, alkoxy en C₁ à C₈, alkyle en C₁ à C₈, N(R₆)₂, NO₂, Br, F, Cl, I, R₁₃CO₂R₇, X(CH₂)ₙR₈, (CH₂)ₘX'R₈, ou X(C(R₆)₂)ₘOR₆ ; R₄ représente l'hydrogène, un groupe OH, alkoxy en C₁ à C₅, N(R₆)₂, Br, F, Cl, I, NHCOCH₃ ou S(O)_{q}-(alkyle en C₁ à C₅) dans lequel le groupe alkyle en C₁ à C₅ peut être non substitué ou substitué avec un groupe OH, méthoxy ou halogéno ; R₆ représente l'hydrogène, un groupe méthyle ou éthyle ; R₇ représente l'hydrogène, un groupe alkyle en C₁ à C₁₀, alcényle en C₂ à C₁₀, ou alcynyle en C₂ à C₈, tous ces groupes pouvant être non substitués ou substitués avec un ou plusieurs groupes OH, N(R₆)₂, CO₂R₁₂, halogéno ou bien R₇ représente un groupe (CH₂)ₙAr dans lequel n est égal à 0 ou 1, et Ar représente un groupe phényle substitué ; R₁₁ représente l'hydrogène, un groupe phényle ou pyridyle, tous ces groupes pouvant être non substitués ou substitués avec un ou deux groupes alkyle en C₁ à C₄ ; un groupe alkyle en C₁ à C₈, alcényle en C₂ à C₈ ou alcynyle en C₂ à C₈, tous ces groupes pouvant être non substitués ou substitués avec un ou plusieurs groupes OH, CH₂OH, N(R₆)₂ ou halogéno ; R₁₂ représente l'hydrogène ou un groupe alkyle en C₁ à C₆ ; R₁₃ représente un groupe phénylène, pyridylène ou alkylène en C₂ à C₁₀, tous ces groupes pouvant être non substitués ou substitués avec un ou plusieurs groupes CO₂R₆, OH, CH₂OH, N(R₆)₂ ou halogéno ; R₁₅ représente l'hydrogène ou un groupe alkyle en C₁ à C₆ ; et (Z) représente un groupe (d).

3. Composé suivant la revendication 2, dans lequel P représente un groupe CO₂H ; R₁ représente l'hydrogène ; R₂ représente un groupe Ar, ledit groupe Ar étant un groupe (a) ou (b) et, dans ledit groupe (a) ou (b), Z₁ et Z₂ représentent indépendamment l'hydrogène, un groupe CO₂R₆, (CH₂)ₙOH, alkyle en C₁ à C₄ ou alkoxy en C₁ à C₆, et A représente un groupe CH₂, et les deux symboles B représentent O ; R₃ représente un groupe Br, Cl, alkoxy en C₁ à C₈ ou X(CH₂)ₙR₈, dans lequel X représente O, n est égal à 0, 1 ou 2, et R₈ est choisi entre des groupes : CO₂H, OH, tétrazolyle facultativement substitué avec un groupe alkyle en C₁ à C₈ ; CONR₇SO₂R₁₁, dans lequel R₇ représente H ou un groupe alkyle en C₁ à C₈, R₁₁ représente un groupe alkyle en C₁ à C₈ ou phényle facultativement substitué avec un groupe Br, Cl, F ou alkyle en C₁ à C₈ ; ou bien R₈ représente un groupe phényle ou pyridyle substitué avec un ou plusieurs groupes Br, Cl, CO₂H, CH₂OH ; R₅ représente un groupe méthoxy ou N(R₆)₂ dans lequel R₆ représente H ou un groupe méthyle ; R₄ représente l'hydrogène ; R₆ représente l'hydrogène, un groupe méthyle ou éthyle ; R₇ représente l'hydrogène, un groupe alkyle en C₁ à C₁₀, alcényle en C₂ à C₁₀, ou alcynyle en C₂ à C₈, tous ces groupes pouvant être non substitués ou substitués avec un ou plusieurs groupes OH, N(R₆)₂, CO₂R₁₂ ou halogéno, ou bien R₇ représente un groupe (CH₂)ₙAr dans lequel n est égal à 0 ou 1 et Ar représente un groupe phényle non substitué ou substitué avec un groupe halogéno ou alkoxy en C₁ à C₅ ; R₁₁ représente l'hydrogène, un groupe phényle ou pyridyle, ledit groupe phényle ou pyridyle pouvant être non substitué ou substitué avec un ou deux groupes alkyle en C₁ à C₄ ; un groupe alkyle en C₁ à C₈, alcényle en C₂ à C₈ ou alcynyle en C₂ à C₈, tous ces groupes pouvant être non substitués ou substitués avec un ou plusieurs groupes OH, CH₂OH, N(R₆)₂ ou halogéno ; R₁₂ représente l'hydrogène ou un groupe alkyle en C₁ à C₆ ; R₁₃ représente un groupe phénylène, pyridylène ou alkylène en C₂ à C₁₀, tous ces groupes pouvant être non substitués ou substitués avec un ou plusieurs groupes CO₂R₆, OH, CH₂OH, N(R₆)₂ ou halogéno ; R₁₅ représente l'hydrogène, un groupe éthyle, isopropyle, n-butyle, cyclopropylméthyle ou cyclopropyléthyle ; et (Z) représente un groupe (d).

4. Composé suivant la revendication 1, choisi entre les suivants :
acide (E)-alpha- [[5-[2- [(2-carboxyphényl)-méthoxy]-4-méthoxyphényl]-éthyl-lH-pyrazole-4-yl]-méthylène] -6-méthoxy-1,3-benzodioxole-5-propanoïque ;
acide (E) -alpha-[[1-allyl-5-[2-[(2-carboxyphényl) -méthoxy]-4-méthoxyphényl]-1H-pyrazole-4-yl]-méthylène]-6-méthoxy-1,3-benzodioxole-5-propanoique ;
acide (E) -alpha- [[1-butyl-5- [2- [(2-carboxyphényl) -méthoxy]-4-chlorophényl] -1H-pyrazole-4-yl]-méthylène] -6-méthoxy-1,3-benzodioxole-5-propanoïque ;
acide (E)-alpha-[[1-butyl-5- [4-méthoxy-2-[[N-(phénylsulfonyl)]carboxamidométhoxy]-phényl] -1H-pyrazole-4-yl] -méthylène] -6-méthoxy-1,3-benzodioxole-5-propanoique ;
acide (E)-3-[1-butyl-5-[2-[(2-carboxyphényl)-méthoxy]-4-méthoxyphényl]-1H-pyrazole-4-yl]-2-[(2,5-diméthoxyphényl) -méthyl]prop-2-énoïque ;
acide (E)-alpha-[[1-butyl-5-[2-[(2-hydroxyméthyl-phényl) -méthoxy]-4-méthoxyphényl]-1H-pyrazole-4-yl]-méthylène]-6-méthoxy-1,3-benzodioxole-5-propanoïque ;
acide (E)-alpha-[[1-butyl-5-[2-[(2-carboxyphényl) -méthoxy] -4-méthoxyphényl] -1H-pyrazole-4-yl] -méthylène] -7-méthoxy-1,4-benzodioxane-6-propanoique ;
acide (E)-alpha-[[5-[2-[(2-carboxyphényl)-méthoxy] -4-méthoxyphényl]-1-cyclopropylméthyl-lH-pyrazole-4-yl]-méthylène]-6-méthoxy-1,3-benzodioxole-5-propanoïque ;
acide (E)-alpha-[[1-(3-butényl)-5-[2-[(2-hydroxyméthylphényl) -méthoxy]-4-méthoxyphényl]-1H-pyrazole-4-yl]-méthylène]-6-méthoxy-1,3-benzodioxole-5-propanoïque ;
acide (E) -alpha- [[5-(2-[(2-carboxyphényl)-méthoxy]-4-méthoxyphényl]-1-cyclopropyléthyl-lH-pyrazole-4-yl] -méthylène] -6-méthoxy-1,3-benzodioxole-5-propanoïque ;
acide (E) -alpha- [[1-butyl-5-[2-[(2-carboxyphénoxy)-méthyl]-4-méthoxyphényl]-1H-pyrazole-4-yl]-méthylène]-6-méthoxy-1,3-benzodioxole-5-propanoïque.

5. Composé suivant la revendication 1, qui est l'acide (E) -alpha-[[1-butyl-5-[2-[(2-carboxyphényl)-méthoxy]-4-méthoxyphényl] -1H-pyrazole-4-yl]-méthylène]-6-méthoxy-1,3-benzodioxole-5-propanoïque.

6. Composé suivant la revendication 5, qui est le sel disodique de cet acide.

7. Composition pharmaceutique comprenant un composé suivant l'une quelconque des revendications 1 à 6 et un support pharmaceutiquement acceptable.

8. Composé suivant l'une quelconque des revendications 1 à 6, destiné à être utilisé comme antagoniste des récepteurs d'endothéline.

9. Utilisation d'un composé de formule (I) suivant l'une quelconque des revendications 1 à 6, dans la production d'un médicament destiné au traitement d'une maladie provoquée par un excès d'endothéline.

10. Utilisation d'un composé suivant la revendication 9, dans lequel la maladie consiste en l'insuffisance rénale ou une maladie vasculaire cérébrale.

11. Utilisation d'un composé suivant la revendication 9 dans la production d'un médicament destiné à la prophylaxie ou au traitement de l'insuffisance rénale induite par un produit de contraste radioactif.

12. Utilisation d'un composé suivant la revendication 9, dans lequel la maladie est l'insuffisance cardiaque congestive.

13. Utilisation d'un composé suivant la revendication 9, dans lequel la maladie consiste en l'angor instable, un angiospasme coronarien ou le sauvetage du myocarde.

14. Utilisation d'un composé suivant la revendication 9, dans la production d'un médicament destiné à la prévention ou au traitement d'une resténose.

15. Utilisation d'un composé suivant la revendication 9, dans laquelle la maladie est l'hypertension pulmonaire.

16. Utilisation d'un composé suivant la revendication 9, dans laquelle la maladie est un ictus ou une hémorragie sous-arachnoïdienne.

17. Procédé pour la préparation d'un composé de formule (Id) par :
(a) réaction d'un composé de formule (II) : 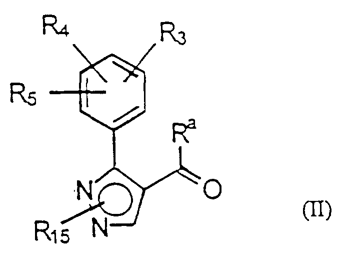 ou d'une de ses formes protégées ou bien d'un de ses précurseurs avec un composé de formule (8) : 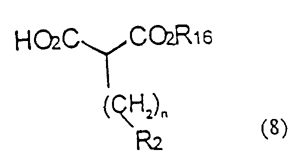 dans laquelle R₂ et R₁₆ répondent aux définitions mentionnées pour la formule (Id) dans la revendication 1 ; avec ensuite, si cela est nécessaire ou désiré :
(b) la conversion d'un composé de formule (Id) en un composé différent de formule (Id), par exemple
(i) lorsque la formule (Id) contient un groupe CO₂R₆, CO₂R₇ ou CO₂R₁₂ dans lequel R₆, R₇ ou R₁₂ représente un groupe alkyle, la conversion en un composé correspondant dans lequel R₆, R₇ ou R₁₂ représente l'hydrogène ;
(ii) lorsque la formule (Id) contient un groupe hydroxy (par exemple dans R₃, R₄ ou R₅), la conversion en un groupe différent, par exemple un groupe (CH₂)Ar dans lequel Ar représente un groupe phényle facultativement substitué, par un procédé bien connu dans ce domaine ; et/ou
(c) la formation d'un sel.
